# EUROPEAN PATENT SPECIFICATION

(11) **EP 0 962 531 B1**
(45) Date of publication and mention of the grant of the patent: **05.08.2009**
(21) Application number: 99104104.7
(22) Date of filing: 10.11.1995
(51) Int. Cl.: C12N 15/24, C07K 14/54

(54) **Interferon-gamma production inducing isolated polypeptide fragment and an isolated DNA fragment encoding said polypeptide**
Isoliertes Polypeptidfragment, das die Interferon-gamma Produktion induziert, und für das Polypeptid kodierende DNA
Fragment isolé polypeptidique induisant la production d'interféron gamma et ADN codant pour celui-ci

(30) Priority: 15.11.1994 JP 30420394; 23.02.1995 JP 5824095; 10.03.1995 JP 7835795; 18.09.1995 JP 26206295; 29.09.1995 JP 27498895
(43) Date of publication of application: 08.12.1999
(62) Divisional of application: 95308055.3
(73) Proprietor: KABUSHIKI KAISHA HAYASHIBARA SEIBUTSU KAGAKU KENKYUJO, Okayama-shi, Okayama (JP)
(72) Inventor: Ushio, Shimpei, Okayama (JP); Torigoe, Kakuji, Okayama (JP); Tanimoto, Tadao, Okayama (JP); Okamura, Haruki, Osaka (JP); Kunikata, Toshio, Okayama (JP); Taniguchi, Mutsuko, Okayama (JP); Kohno, Keizo, Okayama (JP); Fukuda, Shigeharu, Okayama (JP); Kurimoto, Masashi, Okayama (JP)
(74) Representative: Daniels, Jeffrey Nicholas

(56) References cited:
- EP-A- 0 692 536
- WO-A-92/05256
- H. OKAMURA ET AL.: "Cloning of a new cytokine that induces IFN-gamma production by T cells" NATURE, vol. 378, 2 November 1995, LONDON GB, pages 88-91, XP002024313
- DATABASE WPI Section Ch, Week 9347 Derwent Publications Ltd., London, GB; Class B04, AN 93-374598 XP002024314 & JP 05 279 376 A (M SATO) , 26 October 1993

### SEQUENCE LISTING

(1) GENERAL INFORMATION:
   (i) APPLICANT:
      NAME:KABUSHIKI KAISHA HAYASHIBARA SEIBUTSU KAGAKU KENKYUJO
   (ii) TITLE OF INVENTION:INTERFERON-GAMMA PRODUCTION INDUCING
      POLYPEPTIDE, MONOCLONAL ANTIBODY SPECIFIC TO SAID POLYPEPTIDE, AND AGENT FOR SUSCEPTIVE DISEASE
   (iii) NUMBER OF SEQUENCES:9
   (iv) ADDRESS:
      (A) ADDRESSEE:KABUSHIKI KAISHA HAYASHIBARA SEIBUTSU KAGAKU KENKYUJO
      (B) STREET:2-3, 1-CHOME, SHIMOISHII
      (C) CITY:OKAYAMA
      (E) COUNTRY:JAPAN
      (F) POSTAL CODE (ZIP) :700
   (v) COMPUTER READABLE FORM:
      (A) MEDIUM TYPE:Floppy disk
      (B) COMPUTER:IBM PC compatible
      (C) OPERATING SYSTEM:PC-DOS/MS-DOS
   (vii) PRIOR APPLICATION DATA:
      (A1) APPLICATION NUMBER:JP 304,203/94
      (B1) FILING DATE:November 15, 1994
      (A2) APPLICATION REFERENCE NUMBER:10048102
      (B2) FILING DATE:September 18, 1995
      (A3) APPLICATION NUMBER:JP 58,240/95
      (B3) FILING DATE:February 23, 1995
      (A4) APPLICATION NUMBER:JP 78,357/95
      (B4) FILING DATE:March 10, 1995
      (A5) APPLICATION REFERENCE NUMBER:10049202
      (B5) FILING DATE:September 29, 1995
(2)INFORMATION FOR SEQ ID NO:1:
   (i)SEQUENCE CHARACTERISTICS:
      (A)LENGTH:157 amino acids
      (B)TYPE:amino acid
      (D)TOPOLOGY:linear
   (ii)MOLECULE TYPE:peptide
   (xi)SEQUENCE DESCRIPTION:SEQ ID NO:1:
   Tyr Phe Gly Lys Leu Glu Ser Lys Leu Ser Val Ile Arg Asn Leu Asn
   1               5                   10                  15
   Asp Gln Val Leu Phe Ile Asp Gln Gly Asn Arg Pro Leu Phe Glu Asp
               20                  25                  30
   Met Thr Asp Ser Asp Cys Arg Asp Asn Ala Pro Arg Thr Ile Phe Ile
           35                  40                  45
   Ile Ser Met Tyr Lys Asp Ser Gln Pro Arg Gly Met Ala Val Thr Ile
       50                  55                  60
   Ser Val Lys Cys Glu Lys Ile Ser Xaa Leu Ser Cys Glu Asn Lys Ile
   65                  70                  75                  80
   Ile Ser Phe Lys Glu Met Asn Pro Pro Asp Asn Ile Lys Asp Thr Lys
                   85                  90                  95
   Ser Asp Ile Ile Phe Phe Gln Arg Ser Val Pro Gly His Asp Asn Lys
               100                 105                 110
   Met Gln Phe Glu Ser Ser Ser Tyr Glu Gly Tyr Phe Leu Ala Cys Glu
           115                 120                 125
   Lys Glu Arg Asp Leu Phe Lys Leu Ile Leu Lys Lys Glu Asp Glu Leu
       130                 135                 140
   Gly Asp Arg Ser Ile Met Phe Thr Val Gln Asn Glu Asp
   145                 150                 155
(3)INFORMATION FOR SEQ ID NO:2:
   (i)SEQUENCE CHARACTERISTICS:
      (A)LENGTH:471 base pairs
      (B)TYPE:nucleic acid
   (xi)SEQUENCE DESCRIPTION:SEQ ID NO:2:
   TACTTTGGCA AGCTTGAATC TAAATTATCA GTCATAAGAA ATTTGAATGA CCAAGTTCTC  60
   TTCATTGACC AAGGAAATCG GCCTCTATTT GAAGATATGA CTGATTCTGA CTGTAGAGAT 120
   AATGCACCCC GGACCATATT TATTATAAGT ATGTATAAAG ATAGCCAGCC TAGAGGTATG 180
   GCTGTAACTA TCTCTGTGAA GTGTGAGAAA ATTTCAAYTC TCTCCTGTGA GAACAAAATT 240
   ATTTCCTTTA AGGAAATGAA TCCTCCTGAT AACATCAAGG ATACAAAAAG TGACATCATA 300
   TTCTTTCAGA GAAGTGTCCC AGGACATGAT AATAAGATGC AATTTGAATC TTCATCATAC 360
   GAAGGATACT TTCTAGCTTG TGAAAAAGAG AGAGACCTTT TTAAACTCAT TTTGAAAAAA 420
   GAGGATGAAT TGGGGGATAG ATCTATAATG TTCACTGTTC AAAACGAAGA C          471
(4)INFORMATION FOR SEQ ID NO:3:
   (i)SEQUENCE CHARACTERISTICS:
      (A)LENGTH:471 base pairs
      (B)TYPE:nucleic acid
      (C)STRANDEDNESS:double
      (D)TOPOLOGY:linear
   (ii)MOLECULE TYPE:cDNA to mRNA
   (iii)HYPOTHETICAL:No
   (iv)ANTI-SENSE:No
   (vi)ORIGINAL SOURCE:
      (A)ORGANISM:mouse
      (F)TISSUE TYPE:liver
   (ix) FEATURE:
      (A)NAME/KEY:1-471 mat peptide
      (C)IDENTIFICATION METHOD:S
   (xi)SEQUENCE DESCRIPTION:SEQ ID NO:3:
   AAC TTT GGC CGA CTT CAC TGT ACA ACC GCA GTA ATA CGG AAT ATA AAT    48
   Asn Phe Gly Arg Leu His Cys Thr Thr Ala Val Ile Arg Asn Ile Asn
   1               5                   10                  15
   GAC CAA GTT CTC TTC GTT GAC AAA AGA CAG CCT GTG TTC GAG GAT ATG    96
   Asp Gln Val Leu Phe Val Asp Lys Arg Gln Pro Val Phe Glu Asp Met
               20                  25                  30
   ACT GAT ATT GAT CAA AGT GCC AGT GAA CCC CAG ACC AGA CTG ATA ATA   144
   Thr Asp Ile Asp Gln Ser Ala Ser Glu Pro Gln Thr Arg Leu Ile Ile
           35                  40                  45
   TAC ATG TAC AAA GAC AGT GAA GTA AGA GGA CTG GCT GTG ACC CTC TCT   192
   Tyr Met Tyr Lys Asp Ser Glu Val Arg Gly Leu Ala Val Thr Leu Ser
       50                  55                  60
   GTG AAG GAT AGT AAA AYG TCT ACC CTC TCC TGT AAG AAC AAG ATC ATT   240
   Val Lys Asp Ser Lys Xaa Ser Thr Leu Ser Cys Lys Asn Lys Ile Ile
   65                  70                  75                  80
   TCC TTT GAG GAA ATG GAT CCA CCT GAA AAT ATT GAT GAT ATA CAA AGT   288
   Ser Phe Glu Glu Met Asp Pro Pro Glu Asn Ile Asp Asp Ile Gln Ser
                   85                  90                  95
   GAT CTC ATA TTC TTT CAG AAA CGT GTT CCA GGA CAC AAC AAG ATG GAG   336
   Asp Leu Ile Phe Phe Gln Lys Arg Val Pro Gly His Asn Lys Met Glu
               100                 105                 110
   TTT GAA TCT TCA CTG TAT GAA GGA CAC TTT CTT GCT TGC CAA AAG GAA   384
   Phe Glu Ser Ser Leu Tyr Glu Gly His Phe Leu Ala Cys Gln Lys Glu
           115                 120                 125
   GAT GAT GCT TTC AAA CTC ATT CTG AAA AAA AAG GAT GAA AAT GGG GAT   432
   Asp Asp Ala Phe Lys Leu Ile Leu Lys Lys Lys Asp Glu Asn Gly Asp
       130                 135                 140
   AAA TCT GTA ATG TTC ACT CTC ACT AAC TTA CAT CAA AGT               471
   Lys Ser Val Met Phe Thr Leu Thr Asn Leu His Gln Ser
   145                 150                 155
(5) INFORMATION FOR SEQ ID NO:4:
   (i)SEQUENCE CHARACTERISTICS:
      (A)LENGTH:25 amino acids
      (B)TYPE:amino acid
      (D)TOPOLOGY:linear
   (ii)MOLECULE TYPE:peptide
   (v)FRAGMENT TYPE:internal fragment
   (xi)SEQUENCE DESCRIPTION:SEQ ID NO:4:
   Ile Ile Ser Phe Glu Glu Met Asp Pro Pro Glu Asn Ile Asp Asp Ile
   1               5                   10                  15
   Gln Ser Asp Leu Ile Phe Phe Gln Lys
               20                  25
(6)INFORMATION FOR SEQ ID NO:5:
   (i)SEQUENCE CHARACTERISTICS:
      (A)LENGTH:18 amino acids -
      (B)TYPE:amino acid
      (D)TOPOLOGY:linear
   (ii)MOLECULE TYPE:peptide
   (v)FRAGMENT TYPE:internal fragment
   (xi)SEQUENCE DESCRIPTION:SEQ ID NO:5:
   Gln Pro Val Phe Glu Asp Met Thr Asp Ile Asp Gln Ser Ala Ser Glu
   1               5                   10                  15
   Pro Gln
(7)INFORMATION FOR SEQ ID NO:6:
   (i)SEQUENCE CHARACTERISTICS:
      (A)LENGTH:1120 base pairs
      (B)TYPE:nucleic acid
      (C)STRANDEDNESS:double
      (D)TOPOLOGY:linear
   (ii)MOLECULE TYPE:cDNA to mRNA
   (iii)HYPOTHETICAL:No
   (iv)ANTI-SENSE:No
   (vi)ORIGINAL SOURCE:
      (A)ORGANISM:human
      (F)TISSUE TYPE:liver
   (ix)FEATURE:
      (A1)NAME/KEY:1-177 5'-UTR
      (C1) IDENTIFICATION METHOD:S
      (A2)NAME/KEY:178-285 leader peptide
      (C2)IDENTIFICATION METHOD:S
      (A3)NAME/KEY:286-756 mat peptide
      (C3)IDENTIFICATION METHOD:S
      (A4)NAME/KEY:757-1120 3'-UTR
      (C4) IDENTIFICATION METHOD:S
   (xi)SEQUENCE DESCRIPTION:SEQ ID NO:6:
   GCCTGGACAG TCAGCAAGGA ATTGTCTCCC AGTGCATTTT GCCCTCCTGG CTGCCAACTC   60
   TGGCTGCTAA AGCGGCTGCC ACCTGCTGCA GTCTACACAG CTTCGGGAAG AGGAAAGGAA  120
   CCTCAGACCT TCCAGATCGC TTCCTCTCGC AACAAACTAT TTGTCGCAGG AATAAAG     177
   ATG GCT GCT GAA CCA GTA GAA GAC AAT TGC ATC AAC TTT GTG GCA ATG    225
   Met Ala Ala Glu Pro Val Glu Asp Asn Cys Ile Asn Phe Val Ala Met
   1               5                   10                  15
   AAA TTT ATT GAC AAT ACG CTT TAC TTT ATA GCT GAA GAT GAT GAA AAC    273
   Lys Phe Ile Asp Asn Thr Leu Tyr Phe Ile Ala Glu Asp Asp Glu Asn
               20                  25                  30
   CTG GAA TCA GAT TAC TTT GGC AAG CTT GAA TCT AAA TTA TCA GTC ATA    321
   Leu Glu Ser Asp Tyr Phe Gly Lys Leu Glu Ser Lys Leu Ser Val Ile
           35                  40                  45
   AGA AAT TTG AAT GAC CAA GTT CTC TTC ATT GAC CAA GGA AAT CGG CCT    369
   Arg Asn Leu Asn Asp Gln Val Leu Phe Ile Asp Gln Gly Asn Arg Pro
       50                  55                  60
   CTA TTT GAA GAT ATG ACT GAT TCT GAC TGT AGA GAT AAT GCA CCC CGG    417
   Leu Phe Glu Asp Met Thr Asp Ser Asp Cys Arg Asp Asn Ala Pro Arg
   65                  70                  75                  80
   ACC ATA TTT ATT ATA AGT ATG TAT AAA GAT AGC CAG CCT AGA GGT ATG    465
   Thr Ile Phe Ile Ile Ser Met Tyr Lys Asp Ser Gln Pro Arg Gly Met
                   85                  90                  95
   GCT GTA ACT ATC TCT GTG AAG TGT GAG AAA ATT TCA AYT CTC TCC TGT    513
   Ala Val Thr Ile Ser Val Lys Cys Glu Lys Ile Ser Xaa Leu Ser Cys
               100                 105                 110
   GAG AAC AAA ATT ATT TCC TTT AAG GAA ATG AAT CCT CCT GAT AAC ATC    561
   Glu Asn Lys Ile Ile Ser Phe Lys Glu Met Asn Pro Pro Asp Asn Ile
           115                 120                 125
   AAG GAT ACA AAA AGT GAC ATC ATA TTC TTT CAG AGA AGT GTC CCA GGA    609
   Lys Asp Thr Lys Ser Asp Ile Ile Phe Phe Gln Arg Ser Val Pro Gly
       130                 135                 140
   CAT GAT AAT AAG ATG CAA TTT GAA TCT TCA TCA TAC GAA GGA TAC TTT    657
   His Asp Asn Lys Met Gln Phe Glu Ser Ser Ser Tyr Glu Gly Tyr Phe
   145                 150                 155                 160
   CTA GCT TGT GAA AAA GAG AGA GAC CTT TTT AAA CTC ATT TTG AAA AAA    705
   Leu Ala Cys Glu Lys Glu Arg Asp Leu Phe Lys Leu Ile Leu Lys Lys
                   165                 170                 175
   GAG GAT GAA TTG GGG GAT AGA TCT ATA ATG TTC ACT GTT CAA AAC GAA    753
   Glu Asp Glu Leu Gly Asp Arg Ser Ile Met Phe Thr Val Gln Asn Glu
               180                 185                 190
   GAC TAGCTA TTAAAATTTC ATGCCGGGCG CAGTGGCTCA CGCCTGTAAT CCCAGCCCTT  812
   Asp
   TGGGAGGCTG AGGCGGGCAG ATCACCAGAG GTCAGGTGTT CAAGACCAGC CTGACCAACA  872
   TGGTGAAACC TCATCTCTAC TAAAAATACT AAAAATTAGC TGAGTGTAGT GACGCATGCC  932
   CTCAATCCCA GCTACTCAAG AGGCTGAGGC AGGAGAATCA CTTGCACTCC GGAGGTAGAG  992
   GTTGTGGTGA GCCGAGATTG CACCATTGCG CTCTAGCCTG GGCAACAACA GCAAAACTCC 1052
   ATCTCAAAAA ATAAAATAAA TAAATAAACA AATAAAAAAT TCATAATGTG AAAAAAAAAA 1112
   AAAAAAAA                                                          1120
(8)INFORMATION FOR SEQ ID NO:7:
   (i)SEQUENCE CHARACTERISTICS:
      (A)LENGTH:10 amino acids
      (B)TYPE:amino acid
      (D)TOPOLOGY:linear
   (ii)MOLECULE TYPE:peptide
   (v)FRAGMENT TYPE:N-terminal fragment
   (xi)SEQUENCE DESCRIPTION:SEQ ID NO:7:
   Tyr Phe Gly Lys Leu Glu Ser Lys Leu Ser
   1               5                   10
(9)INFORMATION FOR SEQ ID NO:8:
   (i)SEQUENCE CHARACTERISTICS:
      (A)LENGTH:471 base pairs
      (B)TYPE:nucleic acid
      (C)strandedness:double
      (D)TOPOLOGY:linear
   (ii)MOLECULE TYPE:cDNA to mRNA
   (vi)ORIGINAL SOURCE:
      (A)ORGANISM:human
      (B) INDIVIDUAL ISOLATE:liver
   (ix)FEATURE:
      (A)NAME/KEY:mat peptide
      (B)LOCATION:1..471
      (C)IDENTIFICATION METHOD:S
   (xi)SEQUENCE DESCRIPTION:SEQ ID NO:8:
   TAC TTT GGC AAG CTT GAA TCT AAA TTA TCA GTC ATA AGA AAT TTG AAT    48
   Tyr Phe Gly Lys Leu Glu Ser Lys Leu Ser Val Ile Arg Asn Leu Asn
   1               5                   10                  15
   GAC CAA GTT CTC TTC ATT GAC CAA GGA AAT CGG CCT CTA TTT GAA GAT    96
   Asp Gln Val Leu Phe Ile Asp Gln Gly Asn Arg Pro Leu Phe Glu Asp
               20                  25                  30
   ATG ACT GAT TCT GAC TGT AGA GAT AAT GCA CCC CGG ACC ATA TTT ATT   144
   Met Thr Asp Ser Asp Cys Arg Asp Asn Ala Pro Arg Thr Ile Phe Ile
           35                  40                  45
   ATA AGT ATG TAT AAA GAT AGC CAG CCT AGA GGT ATG GCT GTA ACT ATC   192
   Ile Ser Met Tyr Lys Asp Ser Gln Pro Arg Gly Met Ala Val Thr Ile
       50                  55                  60
   TCT GTG AAG TGT GAG AAA ATT TCA AYT CTC TCC TGT GAG AAC AAA ATT   240
   Ser Val Lys Cys Glu Lys Ile Ser Xaa Leu Ser Cys Glu Asn Lys Ile
   65                  70                  75                  80
   ATT TCC TTT AAG GAA ATG AAT CCT CCT GAT AAC ATC AAG GAT ACA AAA   288
   Ile Ser Phe Lys Glu Met Asn Pro Pro Asp Asn Ile Lys Asp Thr Lys
                   85                  90                  95
   AGT GAC ATC ATA TTC TTT CAG AGA AGT GTC CCA GGA CAT GAT AAT AAG   336
   Ser Asp Ile Ile Phe Phe Gln Arg Ser Val Pro Gly His Asp Asn Lys
               100                 105                 110
   ATG CAA TTT GAA TCT TCA TCA TAC GAA GGA TAC TTT CTA GCT TGT GAA   384
   Met Gln Phe Glu Ser Ser Ser Tyr Glu Gly Tyr Phe Leu Ala Cys Glu
           115                 120                 125
   AAA GAG AGA GAC CTT TTT AAA CTC ATT TTG AAA AAA GAG GAT GAA TTG   432
   Lys Glu Arg Asp Leu Phe Lys Leu Ile Leu Lys Lys Glu Asp Glu Leu
       130                 135                 140
   GGG GAT AGA TCT ATA ATG TTC ACT GTT CAA AAC GAA GAC               471
   Gly Asp Arg Ser Ile Met Phe Thr Val Gln Asn Glu Asp
   145                 150                 155
(10)INFORMATION FOR SEQ ID NO:9:
   (i)SEQUENCE CHARACTERISTICS:
      (A)LENGTH:11
      (B)TYPE:amino acid
      (D)TOPOLOGY:linear
   (ii)MOLECULE TYPE:peptide
   (v)FRAGMENT TYPE:N-terminal fragment
   (xi)SEQUENCE DESCRIPTION:SEQ ID NO:9:
   Met Tyr Phe Gly Lys Leu Glu Ser Lys Leu Ser
   1               5                   10

## Description

IFN-γ is a protein which has antiviral-, antioncotic- and immunoregulatory-activities, and is produced by immunocompetent cells stimulated with antigens or mitogens. Because of these biological activities, IFN-γ is expected for use as an antitumor agent from the beginning of the discovery, and studied energetically on clinical trials as a therapeutic agent for malignant tumors in general including brain tumors. IFN-γ preparations now commercially available are roughly classified into 2 groups, i.e. natural IFN-γs produced by immunocompetent cells and recombinant IFN-γs produced by transformants prepared by introducing into microorganisms of the species *Escherichia coli* DNAs which encode the natural IFN-γs. In the above clinical trials, either of such IFN-γs is administered to patients as an "exogenous IFN-γ".

Among these IFN-γs, the natural IFN-γs are usually produced by culturing established immunocompetent cells in nutrient culture media supplemented with IFN-γ inducers to produce the IFN-γs, and purifying the produced IFN-γs. It is known that the type of IFN-γ inducers greatly influences on the IFN-γ production yield, the facilitation of the IFN-γ purification, and the safeness of the final products. Generally, mitogens such as concanavalin A (Con A), *Lens culinaris*, *Phytolacca americana*, endotoxin and lipopolysaccharide are used. These mitogens, however, have problems of their molecular- and quality-diversities depending on their origins and purification methods, as well as having difficulties of yielding in a desired amount and with a constant IFN-γ inducibility. In addition, most of these mitogens induce unfavorable side effects when administered to living bodies, and some of them even show toxicity. Therefore, it is substantially difficult to induce the IFN-γ production by the direct administration of such mitogens to living bodies.

The present inventors found in mouse liver a substance which induces the IFN-γ production through their researches on cytokines produced by mammalian cells. They isolated the substance by using a variety of purification methods comprising column chromatography as a main technique, studied the properties and features, and revealed that the reality is a protein having the following physicochemical properties:
(1) Molecular weight
   Exhibiting a molecular weight of 19,000±5,000 daltons on sodium dodecyl polyacrylamide gel electrophoresis (SDS-PAGE);
(2) Isoelectric point (pI)
   Exhibiting an isoelectric point of 4.8±1.0 on chromatofocusing;
(3) Partial amino acid sequence
   Having the partial amino acid sequences in SEQ ID NOs:4 and 5; and
(4) Biological activity
   Inducing the IFN-γ production by immunocompetent cells.

It can be concluded that the reality is a novel substance because no protein with these physicochemical properties has been known. The present inventors continued studies on mouse liver cells and have found that the DNA of the substance consists of 471 base pairs and encodes the amino acid sequence in SEQ ID NO:3.

Based on these findings, the present inventors further continued studies on human liver cells and have obtained a DNA which encodes another novel substance that induces the IFN-γ production by immunocompetent cells. They revealed that the reality is a polypeptide and then decoded its DNA to find that it has the amino acid sequence in SEQ ID NO:1.

They introduced the DNA into *Escherichia coli* to express the polypeptide and to produce it in the culture in a considerably high yield.

As is described above, the polypeptide has a property of inducing the IFN-γ production by immunocompetent cells, and is expected to be used in a variety of fields as an IFN-γ inducer, antiviral agent, antitumor agent, antibacterial agent, immunoregulatory agent, and blood platelet enhancing agent. In general, the development of methods for efficiently purifying biologically active polypeptides into ones with a relatively-high purity, and for assaying many samples simultaneously are inevitably required when the polypeptides should be incorporated into pharmaceuticals. Although the most suitable material enabling these purification and assay is a monoclonal antibody, none of which is specific to the polypeptide has been obtained.

Recently, some pharmaceuticals, which contain as an effective ingredient cytokines such as interferon-α, interferon-β, TNF-α, TNF-β, interleukin 2 and interleukin 12, as well as IFN-γ, were developed and others are under exploitation for their actual use. These pharmaceuticals can be used as an antitumor agent, antiviral agent, antiseptic, and immunoregulatory agent, and, if necessary, they can be used along with other medicaments.

Unlike chemically synthesized pharmaceuticals, the aforesaid pharmaceuticals have as the greatest feature a character of being readily administered to patients for a relatively-long period of time without inducing serious side effects, but have demerits that their therapeutic effects are generally relatively-low, and they could not substantially remit or cure diseases if used alone, varying dependently on the types of diseases and symptoms to be treated. Therefore, such pharmaceuticals are now used as a supplemental agent for chemically synthesized agents in the treatment of serious diseases such as malignant tumors, or used as a means to prolong patients' life.

The present invention provides a composition for specifically reacting with a polypeptide of human origin, which comprises as an effective ingredient a monoclonal antibody recognizing the polypeptide along with a carrier of the monoclonal antibody; said polypeptide inducing the IFN-gamma production by immunocompetent cells and comprising the amino acid sequence of SEQ ID No: 1 (where the symbol "Xaa" means "isoleucine" or "threonine").

The present invention also provides a method for specifically reacting a polypeptide of human origin with a monoclonal antibody, which comprises a step of contacting the polypeptide with a monoclonal antibody recognizing the polypeptide; said polypeptide inducing the IFN-gamma production by immunocompetent cells and comprising the amino acid sequence of SEQ ID No: 1 (where the symbol "Xaa" means "isoleucine" or "threonine").

Described herein is an isolated peptide fragment of a human polypeptide, said human polypeptide having an amino acid sequence of SEQ ID NO:1, where Xaa at amino acid residue 73 of SEQ ID NO:1 is isoleucine or threonine, and inducing IFN-γ production by immunocompetent cells.

Described herein is an isolated collection of peptide fragments which are contained in a human polypeptide, said human polypeptide having an amino acid sequence of SEQ ID NO:1, where Xaa at amino acid residue 73 of SEQ ID NO:1 is isoleucine or threonine, and inducing IFN-γ production by immunocompetent cells.

Described herein is an isolated DNA fragment of a DNA of SEQ ID NO:2, said DNA of SEQ ID NO:2 encoding a human polypeptide inducing IFN-γ production by immunocompetent cells.

Described herein is an isolated collection of DNA fragments which are contained in a DNA of SEQ ID NO:2, said DNA of SEQ ID NO:2 encoding a human polypeptide having a nucleotide sequence of SEQ ID NO:1 and inducing IFN-γ production by immunocompetent cells.

The invention will now be described in further detail, by way of example only, with reference to the accompanying drawings, in which:
FIG.1 is an HPLC elution pattern of a peptide fragment obtained by trypsinizing a protein derived from mouse liver.
FIG.2 is a figure of the structure of the present recombinant DNA pHIGIF.
FIG.3 is a figure of the structure of recombinant DNA pKGFHH2.
FIG.4 is a figure of the Western blotting which shows the reactivity of the present purified polypeptide and human interleukin 12 with the present monoclonal antibody H-1mAb.
   HIGIF cDNA : cDNA which encodes the present polypeptide
   KGFHH2 cDNA : cDNA encoding the present polypeptide
   Ptac : tac promoter
   rrnBT1T2 : terminator of ribosome RNA operon
   GST : glutathione S transferase gene
   AmpR : ampicillin resistant gene
   pBR322ori : replication initiation site of *Escherichia coli*

A polypeptide according to the present description has an amino acid sequence which differs from those of conventional polypeptides, and induces the IFN-γ production when allowed alone or together with a cofactor to act on immunocompetent cells.

A DNA according to the present description expresses the production of the present polypeptide by introducing the DNA into a self-replicable vector to form a recombinant DNA, and, usually, introducing the recombinant DNA into a host capable of proliferating without difficulty but incapable of producing the polypeptide.

Generally, a replicable recombinant DNA according to the present description expresses the production of the present polypeptide by introducing it into a host capable of proliferating without difficulty but incapable of producing the polypeptide.

A transformant produces the present polypeptide when cultured.

The present polypeptide is readily obtained in a desired amount by culturing the transformant according to the present process.

The present description is based on the finding of a novel polypeptide which induces the IFN-γ production by immunocompetent cells. During studies on cytokines produced from mammalian cells, the present inventors found that there exists in mouse liver a novel protein capable of inducing the IFN-γ production. They isolated the protein by using two or more purification methods comprising column chromatography mainly and determined for the partial amino acid sequence. Based on the sequence, they chemically synthesized a primer by using as a template a mRNA isolated from mouse liver cells, and treated the protein with transcription-polymerase chain reaction (RT-PCR) in the presence of the primer to collect DNA fragments which partially encode the protein. By using the DNA fragments as a probe, they energetically studied a cDNA library which was alternatively prepared from the mRNA, and obtained a DNA fragment consisting of 471 base pairs and having the base sequence of SEQ ID NO:3. The decoding of the base sequence revealed that the protein, isolated from mouse liver, consists of 157 amino acids and has an amino acid sequence in SEQ ID NO:3, where the symbol "Xaa" means "methionine" or "threonine".

Based on these findings, the present inventors further studied the mRNA derived from human liver cells, and have found that there exists a new gene which encodes a polypeptide which induces the IFN-γ production by immunocompetent cells. The gene contains the base sequence in SEQ ID NO:2, and the decoding thereof revealed that it encodes a polypeptide having the amino acid sequence in SEQ ID NO:1 where the symbol "Xaa" means "isoleucine" or "threonine".

The techniques used to reveal the amino acid sequence and the base sequences in SEQ ID NOs:1 and 2 are summarized in the below:
(1) A protein, which induces the IFN-γ production by immunocompetent cells, was isolated from mouse liver cells and highly purified by combining conventional purification methods comprising chromatography as a main technique;
(2) The resultant purified protein was digested with trypsin, and 2 polypeptide fragments were isolated from the resultant mixture and determined for amino acid sequence;
(3) From mouse liver cells, a mRNA was collected and subjected as a template to the reverse transcription-polymerase chain reaction (RT-PCR) to obtain DNA fragments in the presence of an oligonucleotide as a primer which had been chemically synthesized based on the above partial amino acid sequence. The fragments were screened by using an oligonucleotide as a probe which had been chemically synthesized based on these partial amino acid sequences, followed by collecting a DNA fragment which partially encodes the protein;
(4) A cDNA library was labeled and hybridized with the resultant cDNA library prepared with the mRNA as a template, followed by selecting a transformant which exhibited a strong hybridization;
(5) A cDNA was isolated from the transformant, and the base sequence was determined and decoded. The comparison of the decoded amino acid sequence and the partial amino acid sequence revealed that the protein has the amino acid sequence in SEQ ID NO:3, and, in mice, the base sequence in SEQ ID NO:3 encodes the amino acid sequence;
(6) A DNA fragment having the base sequence in SEQ ID NO:3 was prepared, labeled and hybridized with a cDNA library which had been prepared by using as a template mRNA derived from human liver cells, followed by selecting a transformant which exhibited a strong hybridization; and
(7) The cDNA was prepared from the transformant, determined for base sequence and decoded, revealing that the present polypeptide, a human polypeptide, includes those with the amino acid sequence in SEQ ID NO:1 encoded by the base sequence in SEQ ID NO:2.

Through long term research, the present inventors have found a polypeptide which induces the IFN-γ production by immunocompetent cells, and, as is evident from SEQ ID NO:1, it differs from conventionally known polypeptides. The present polypeptide includes natural and recombinant polypeptides as long as they have the amino acid sequence in SEQ ID NO:1 or homologous ones thereunto. Variants, which have homologous amino acid sequences to the one in SEQ ID NO:1, can be obtained by replacing one or more amino acids in SEQ ID NO:1 with other amino acids without alternating the inherent biological activity of the present polypeptide. Depending on hosts into which DNAs, even when used the same DNAs, are introduced and on the components and the conditions of cultivation temperature and pH for transformants containing the DNA, it may be formed variants which lack one or more amino acids near to the N- and/or C-termini in SEQ ID NO:1, or contain additionally one or more amino acids near to the N-terminus in SEQ ID NO:1 through the modification of internal enzymes of the hosts after the DNA expression, while keeping the inherent biological properties of the polypeptide. The present polypeptide includes such variants as long as they induce the IFN-γ production by immunocompetent cells.

The present polypeptide can be prepared by culturing in nutrient culture media transformants which contain DNAs encoding the polypeptide, and collecting the produced polypeptide from the resultant cultures. The transformants usable in the present invention can be obtained, for example, by introducing into hosts DNAs having the base sequence in SEQ ID NO:2, homologous base sequences thereunto, and complementary ones to these base sequences. One or more bases in those base sequences can be replaced with other bases by means of the degeneracy of genetic code without alternating the amino acid sequence of the present polypeptide. To express the production of the polypeptide in hosts by using such DNAs, one or more bases in base sequences which encode the present polypeptide or its variants can be replaced with other bases.

Any DNA can be used in the present description as long as it has one of those base sequences independently of their origin, i.e. those from natural sources or artificially synthesized ones. The natural sources include, for example, human liver cells from which the gene, containing the DNA with the base sequence in SEQ ID NO:6, is obtainable.

The preparation procedure is as follows: Fractionate a commercially available human liver mRNA supplemented with poly(A) on a sucrose gradient buffer to isolate the purified mRNA. Allow a reverse transcriptase and a polymerase to act on the mRNA as a template to form a double-stranded cDNA, introduce the cDNA into an appropriate self-replicable vector, and introduce the resultant recombinant DNA into an appropriate host such as *Escherichia coli*. Culture the resultant transformant in a nutrient culture medium, and collect the proliferated transformants containing the DNA encoding the present polypeptide by the colony hybridization method. The DNA according to the present description is obtainable by treating the transformants with conventional methods. To artificially produce the present DNA, for example, it is prepared by the chemical synthesis based on the base sequence in SEQ ID NO:2, or by introducing a DNA which encodes the amino acid sequence in SEQ ID NO:1 into an appropriate vector to form a recombinant DNA, introducing the recombinant DNA into an appropriate host, culturing the resultant transformant in a nutrient culture medium, isolating the proliferated cells from the culture, and collecting plasmids containing the objective DNA from the cells.

Generally, the DNA is introduced into hosts in the form of a recombinant DNA. Such a recombinant DNA usually contains the DNA and a self-replicable vector, and it can be readily prepared by recombinant DNA technology in general if only the DNA is in hand. Examples of such self-replicable vector are plasmid vectors such as pKK223-2, pGEX-2T, pRL-λ, pBTrp2 DNA, pUB110, YEp13, Ti plasmid, Ri plasmid and pBI121. Among these vectors, pKK223-2, pGEX-2T, pRL-λ, pBTrp2 DNA, pUB110 and YEp13 are suitably used when the present DNA is expressed in procaryotes such as yeasts and other microorganisms of the species *Escherichia coli* and *Bacillus subtilis*, while Ti plasmid, Ri plasmid and pBI121 are suitably used for the expression in animal and plant cells.

To introduce the present DNA into these vectors, conventional methods used in this field can be arbitrarily used: Genes containing the present DNA and self-replicable vectors are cleaved with restriction enzymes and/or ultrasonic, and the resultant DNA fragments and vector fragments are ligated. To cleave genes and vectors, restriction enzymes which specifically act on nucleotides, more particularly, type II restriction enzymes such as *Sau* 3AI, *Eco* RI, *Hind* III, *Bam* HI, *Sal* I, *Xba* I, *Sac* I and *Pst* I, facilitate the ligation of DNA fragments and vector fragments. To ligate DNA fragments and vector fragments, they are, if necessary, first annealed, then treated with a DNA ligase *in vivo* or *in vitro*. The recombinant DNAs thus obtained can be readily introduced into appropriate hosts, and this enables the limitless replication of the DNAs by culturing the transformants.

The recombinant DNAs usable in the present description can be introduced into appropriate hosts such as yeasts and other microorganisms of the species *Escherichia coli* and *Bacillus subtilis*: When microorganisms of the species *Escherichia coli* are used as a host, they are cultured in the presence of the recombinant DNAs and calcium ions, and the competent cell method and the protoplast method are used when microorganisms of the species *Bacillus subtilis* are used as a host. To clone the objective transformants, they are selected by the colony hybridization method or by culturing all the transformants in nutrient culture media, and selecting those which produce polypeptides capable of inducing the IFN-γ production by immunocompetent cells.

The transformants thus obtained produce the present polypeptide intracellularly or extracellularly when cultured in nutrient culture media. Examples of such nutrient culture media are those in the form of liquid in general which contain carbon sources, nitrogen sources, and minerals, as well as amino acids and/or vitamins as a micronutrient. The carbon sources usable in the present description include saccharides such as starch, starch hydrolysates, glucose, fructose and sucrose. The nitrogen sources usable in the present invention include nitrogen containing organic- and inorganic-compounds such as ammonia and their salts, urea, nitrates, peptone, yeast extract, defatted soy bean, corn steep liquor, and beef extract. Transformants are inoculated into nutrient culture media and incubated at a temperature of 25-65°C and at a pH of 5-8 for about 1-10 days under aerobic conditions by the agitation-aeration method, etc., to obtain cultures containing the present polypeptide. Although the cultures can be used intact as an IFN-γ inducer, they are, if necessary, subjected to ultrasonication and/or cell lysis enzymes to disrupt cells, followed by filtering or centrifuging the resultant suspensions to remove intact cells and cell debris, and further purifying the resultant supernatants containing the present polypeptide. The purification methods usable in the present description are, for example, those which are generally used in this field to purify biologically active substances, i.e. concentration, salting out, dialysis, separatory sedimentation, gel filtration chromatography, ion-exchange chromatography, hydrophobic chromatography, affinity chromatography, chromatofocusing, gel electrophoresis, and isoelectrophoresis, and, if necessary, two or more of them can be used in combination. The resultant purified solutions containing the present polypeptide can be concentrated and/or lyophilized into liquids or solids to meet to final uses.

As is described above, the present polypeptide has an activity of inducing the IFN-γ production by immunocompetent cells. Because of this, the present polypeptide can be arbitrarily used as therapeutic and/or prophylactic agents, for example, those for virus diseases such as AIDS and condyloma acuminatum; malignant tumors such as renal cancer, granuloma, mycosis fungoides and cerebral tumor; and immune disorders such as articular rheumatism and allergy.

The present polypeptide is allowed to coexist in nutrient culture media to induce the IFN-γ production by immunocompetent cells, or directly administered to mammals for the treatment and/or the prevention of IFN-γ susceptive diseases. In the former, leukocytes separated from mammalian peripheral blood, or established immunocompetent cells such as HBL-38 cells, Mo cells, Jurkat cells, HuT78 cells, EL4 cells and L12-R4 cells are suspended in nutrient culture media containing about 0.1 ng to about one µg per ml, preferably, about 1-100 ng per ml of the present polypeptide to induce the IFN-γ production. If necessary, such nutrient culture media can be supplemented with T-cell stimulants such as mitogen, interleukin 2, and anti-CD 3 antibody, and the cells are cultured at a temperature of about 30-40°C and at a pH of about 5-8 for about 1-100 hours while the media were replacing with fresh ones. IFN-γ can be obtained from the resultant cultures by one or more conventional methods generally used for purifying biologically active substances, for example, concentration, salting out, dialysis, separatory sedimentation, gel filtration chromatography, ion-exchange chromatography, chromatofocusing, gel electrophoresis, and isoelectrophoresis.

To treat and/or prevent IFN-γ susceptive diseases, the present IFN-γ inducing agent is directly administered to mammals: For example, IFN-γ inducing agents are orally administered to mammals after formulated into appropriate forms, or injected to the mammals intradermally, subcutaneously, muscularly, intravenously or peritoneally. The mammals, which can be administered with the present polypeptide, are not restricted to human, and include other animals such as mouse, rat, hamster, rabbit, dog, cat, caw, horse, coat, sheep, pig and monkey. Since the present polypeptide has a strong IFN-γ inducibility and an extremely-low toxicity, it readily induces the IFN-γ production with only a small amount without causing serious side effects even when administered to the mammals in a relatively-high dose. Thus, the present polypeptide advantageously induces a desired amount of IFN-γ smoothly without strictly controlling the dose level. It goes without saying that the present polypeptide fulfills the safeness required for a pharmaceutical.

The composition comprising a monoclonal antibody according to the present invention specifically reacts with a polypeptide having the amino acid sequence of SEQ ID NO:1.

The hybridoma according to the present description produces the monoclonal antibody when cultured *in vitro*.

The preparation of the monoclonal antibody according to the present description facilitates the production of the antibody in a desired amount.

The purification method of the polypeptide according to the present description efficiently recovers it with a relatively high quality from a mixture containing the polypeptide and impurities.

In the detection method according to the present description only the polypeptide immunologically reacts in samples. When the immunoreaction level is measured by an appropriate technique, the polypeptide can be qualitatively or quantitatively assayed.

The monoclonal antibody according to the present description includes those in general which are specific to the polypeptide having the amino acid sequence in SEQ ID NO:1 or homologous ones thereunto including at least the first ten amino acids as depicted in SEQ ID NO:1, independently of their source, origin and class. The homologous amino acids include those which are obtained by replacing one or more amino acids in SEQ ID NO:1 with other amino acids, by adding one or more amino acids to the N- and/or C-termini in the amino acid sequence of SEQ ID NO:1, or by losing one or more amino acids in the N- and/or C-termini of the amino acid sequence in SEQ ID NO:1, while substantially not losing the IFN-γ production inducing activity for immunocompetent cells.

The monoclonal antibody usable in the composition and method of the present invention can be obtained by using the polypeptide or its antigenic fragments: For example, the antibody can be obtained by preparing hybridomas using mammalian cells capable of infinite proliferation and antibody-producing cells collected from mammals immunized with the fragments, selecting clones of hybridomas capable of producing the monoclonal antibody, and culturing the clones *in vivo* or *in vitro*.

The polypeptide as an antigen can be obtained by culturing transformants into which a DNA encoding the amino acid sequence in SEQ ID NO:1 and or a homologous one was introduced, and, generally, they are used intact or in a partially purified form. The antigenic fragments can be prepared by chemically or enzymatically hydrolyzing a wholly or partially purified polypeptide, or synthesized by peptide synthesis based on the amino acid sequence in SEQ ID NO:1.

The immunization method usable includes conventional ones: For example, antigens alone or in combination with adequate adjuvants are injected into mammals intravenously, intradermally, subcutaneously or intraperitoneally, and they are fed for a prescribed period. Any mammal can be used without special restriction as long as desired antibody-producing cells can be obtained independently of animal's species, weight and sex. In general, rodents such as rats, mice and hamsters are used, and from which the most suitable animal is selected while evaluating the compatibility with the above mammalian cells capable of infinite proliferation. Depending on the species and weight of the animals used, the total dose of the antigens is generally in the range of about 5-500 µg per animal and administered to 2-5 times at an interval of 1-2 weeks. On 3-5 days after the final administration, the animal's spleen is extracted and dispersed into a suspension of spleen cells as an antibody-producing cell.

The antibody-producing cells and the mammalian cells obtained in the above are fused into a cell fusion mixture containing the objective hybridomas. The mammalian cells capable of infinite proliferation include cell strains from mouse myeloma such as P3-NS1-Ag4-1 cells (ATCC TIB18), P3-X63-Ag8 cells (ATCC TIB9), SP2/O-Ag14 cells (ATCC CRL1581), and mutants thereof. The cell fusion method usable includes conventional ones using an electric pulse and a cell fusion-accelerator such as polyethylene glycol and sendai virus (HVJ): For example, antibody-producing cells and such mammalian cells are suspended in a ratio of about 1:1 to 1:10 in fusion media containing fusion accelerators, and incubated at about 30-40°C for about 1-5 min. Conventional media such as minimum essential medium (MEM), RPMI 1640 medium, and Iscove's Modified Dulbecco's Medium (IMDM) are preferably used as a fusion medium without addition of serums such as calf serum.

To select the objective hybridomas, the resultant cell fusion mixture was transferred to selection media such as HAT medium, and incubated at about 30-40°C for about 3 days to 3 weeks to die cells except for the hybridomas. The hybridomas were cultured in usual manner, and antibodies secreted in the cultures were assayed for reactivity with the polypeptide. Examples of such an assay are conventional ones for detecting antibodies such as an enzyme immunoassay, radioimmunoassay, and bioassay. For example, "Tan-Clone-Kotai-Jikken-Manual (Experimental Manual for Monoclonal Antibody)", edited by Sakuji TOYAMA and Tamie ANDO, published by Kodansha Scientific, Ltd., Tokyo, Japan, pp.105-152 (1991) describes a variety of them. Hybridomas, which produce antibodies that are specific to the polypeptide, are readily cloned by limiting dilution to obtain the hybridoma according to the present invention.

The monoclonal antibody usable in the composition and method of the present invention can be obtained by culturing the hybridoma *in vivo*, i.e. in animals, or *in vitro*. For the culture conventional methods for culturing mammalian cells can be used: For example, in case of *in vivo* culture, the monoclonal antibody is collected from the animals' ascites and/or blood. Hybridomas H-1 and H-2 as described in the below have an enhanced producibility of the monoclonal antibody and have a character of being readily cultured *in vivo* and *in vitro*. Conventional methods used to purify antibodies in general can be used to collect the monoclonal antibody from the cultures, and animals' ascites and blood. Examples of such include salting out, dialysis, filtration, concentration, centrifugation, separatory sedimentation gel filtration chromatography, ion-exchange chromatography, affinity chromatography, high-performance liquid chromatography (HPLC), gel electrophoresis, and isoelectrophoresis, and, if necessary, two or more of them can be used in combination. The resultant purified monoclonal antibodies can be concentrated or dried into products in the form of a liquid or a solid to meet to their final use.

The present monoclonal antibody is extremely useful for purifying the present polypeptide on immunoaffinity chromatography. Such a purification technique comprises contacting the monoclonal antibody with a mixture containing the polypeptide and impurities such as proteins other than the polypeptide to adsorb the polypeptide on the antibody, and desorbing the polypeptide from the antibody. These steps are generally carried out in an aqueous system. The monoclonal antibody is generally used in an immobilized form to gel water-insoluble carriers which are packed in cylindrical columns. Cultures of transformants or their partially purified products are fed to the columns to substantially adsorb the polypeptide on the monoclonal antibody. The polypeptide readily desorbs from the antibody by alternating the pH around the antibody. For example, in the case of using a monoclonal antibody of the class IgG, the adsorbed polypeptide desorbs and elutes from the columns at an acidic pH, usually, a pH of 2-3, while in the case of using a monoclonal antibody of the class IgM, the polypeptide desorbs and elutes from the columns at an alkaline pH, usually, a pH of 10-11.

The purification method attains a relatively-high purification level of the polypeptide with only the minimum labor cost and time. As is described above, the polypeptide has an activity of inducing the IFN-γ production by immunocompetent cells, and the purified polypeptide can be used as an IFN-γ inducer for cell culture to produce IFN-γ, and used in the treatment and/or the prevention of virus diseases such as AIDS and condyloma, malignant tumors such as renal cancer, granuloma, mycosis fungoides, and cerebral tumor, and immune diseases such as articular rheumatism and allergy. If the polypeptide has an activity of enhancing the cell cytotoxicity of killer cells, it can be used together with interleukin 2 and/or tumor necrosis factor to improve the therapeutic effect and reduce the side effects in the treatment of adoptive immunity for malignant tumors including solid tumors such as lung cancer, renal cancer, and breast cancer.

The monoclonal antibody has a relatively-wide applicability to a variety of fields which require the detection of the polypeptide. When used in labelled immunoassays such as radioimmunoassay, enzyme immunoassay, and fluorescent immunoassay, the monoclonal antibody can qualitatively and quantitatively detect the polypeptide in samples instantly and accurately. In such assays, the monoclonal antibody is labelled, for example, with radioisotopes, enzymes and/or fluorescent substances prior to use. The antibody specifically reacts with the polypeptide to exhibit an immunoreaction, and accurately detects only a slight amount of the polypeptide in samples by measuring the level of the immunoreaction for these labelled substances. As compared with bioassay, labelled immunoassay has the following features: It can assay many samples simultaneously, reduce the assaying time and labor cost, and provide data in a relatively high accuracy. Thus, the present detection method is useful for controlling the production steps of the polypeptide and for the quality control of the final products. Although the present description does not describe in detail the techniques for labelling monoclonal antibody or labelling assay because it does not in itself relate to such an invention, these techniques are described in detail in "Enzyme Immunoassay", edited by P. Tijssen, translated by Eiji ISHIKAWA, published by Tokyo-Kagaku-Dojin, pp.196-348 (1989).

The present agent for susceptive diseases induces the IFN-γ production by immunocompetent cells when administered to human, and exerts a therapeutic and/or prophylactic effect on IFN-γ susceptive diseases. When the polypeptide has an activity of enhancing the cytotoxicity of killer cells or of inducing the formation of killer cells, it exerts a strong effect in the treatment of serious diseases including malignant tumors.

The isolated fragment polypeptide used in the present description has the amino acid sequence in SEQ ID NO:1 (where the symbol "Xaa" at amino acid residue 73 is "isoleucine" or "threonine") and induces the IFN-γ production by immunocompetent cells. Examples of a homologous amino acid sequence include those which correspond to the amino acid sequence in SEQ ID NO:1 wherein one or more amino acids are replaced with other amino acids, to that wherein one or more amino acids are added to the N- and/or C-termini, and to that wherein one or more amino acids in the N- and/or C-termini are defective. Any polypeptides, for example, those isolated from natural sources by cell culture and those artificially synthesized by recombinant DNA technology and peptide synthesis, can be used in the present description as long as they have of the required amino acid sequences and properties.

With economical view point, recombinant DNA technology is advantageously used in the present description: According to the technology, DNAs encoding those amino acid sequences are introduced into appropriate hosts derived from microorganisms and animals to obtain transformants which are then in usual manner cultured in nutrient culture media, and the resultant cultures are purified on conventional techniques used for purifying cytokines to obtain the objective polypeptide.

As is described above, the polypeptide has a property of inducing the IFN-γ production by immunocompetent cells. When administered to human, the present agent for susceptive diseases induces the IFN-γ production by immunocompetent cells in the body, and exerts a satisfactory therapeutic and/or prophylactic effect on IFN-γ susceptive diseases. The polypeptide having the amino acid sequence in SEQ ID NO:1 has properties of enhancing the cytotoxicity of killer cells such as NK cells, LAK cells (lymphokine-activating killer cells), cytotoxic T-cells, and inducing the formation of the killer cells, as well as having a property of inducing the IFN-γ production by immunocompetent cells, so that the killer cells treat and/or prevent the polypeptide-susceptive diseases. Thus, the wording "susceptive diseases" as referred to in the present specification means diseases in general which include IFN-γ susceptive diseases and those can be directly or indirectly treated and/or prevented by IFN-γs and/or killer cells: For example, viral diseases such as hepatitis, herpes syndrome, condyloma, and AIDS; bacterial diseases such as Candidiasis and malaria; solid malignant tumors such as renal cancer, mycosis fungoides, and chronic granulomatous disease; blood cell malignant tumors such as adult T cell leukemia, chronic myelogenous leukemia, and malignant leukemia; and immune diseases such as allergy and rheumatism. When the polypeptide is used together with interleukin 3, it exerts a strong effect on the treatment or the remission of leukemia and myeloma, as well as leukopenia and thrombopenia induced by radiations and chemotherapeutic agents to treat malignant tumors.

The present agent can be used widely in the treatment and/or prevention of the aforesaid susceptive diseases as an antitumor agent, antiviral agent, antiseptic, immunotherapeutic agent, platelet-increasing agent, and leukocyte-increasing agent. Although it varies dependently on the types of agents used for such purposes and susceptive diseases to be treated, the present agent is generally processed into an agent in the form of a liquid, paste or solid which contains the polypeptide in an amount of 0.000001-100 w/w %, preferably, 0.0001-0.1 w/w %, on a dry solid basis (d.s.b.).

The present agent can be used intact or processed into compositions by mixing with a physiologically-acceptable carrier, adjuvant, excipient, diluent, and/or stabilizer such as serum albumin, gelatin, saccharides including maltose and trehalose, etc., and, if necessary, further mixing with one or more other biologically-active substances such as interferon-α, interferon-β, interleukin 2, interleukin 3, interleukin 12, TNF-α, TNF-β, carboquone, cyclophosphamide, aclarubicin, thiotepa, busulfan, ancitabine, cytarabine, 5-fluorouracil, 5-fluoro-1-(tetrahydro-2-furyl)uracil, methotrexate, actinomycin D, chromomycin A3, daunorubicin, doxorubicin, bleomycin, mitomycin C, vincristine, vinblastine, L-asparaginase, radio gold colloidal, Krestin^{®}, picibanil, lentinan, and Maruyama vaccine. Among these combinations, the one consisting of the polypeptide and interleukin 2 is specifically useful because the interleukin 2 acts as a cofactor for the polypeptide when the polypeptide induces the IFN-γ production by immunocompetent cells. The combination use of the polypeptide and a natural or recombinant human interleukin 2 induces a prescribed level of IFN-γ production even when the polypeptide does not substantially induce the IFN-γ production by immunocompetent cells.

The combination use of the polypeptide and interleukin 12 attains a greater level of IFN-γ inducibility which could not be readily attained by the sole use of them.

The present agent for susceptive diseases includes those in a unit dose form which means a physically separated and formed medicament suitable for administration, and contains the polypeptide in a daily dose or in a dose from 1/40 to several folds (up to 4 folds) of the daily dose. Examples of such medicaments are injections, liquids, powders, granules, tablets, capsules, sublinguals, ophthalmic solutions, nasal drops, and suppositories.

The present agent can be orally or parenterally administered to patients, and, as described in the below, it can be used to activate antitumor cells *in vitro*. In both administrations, the agent exerts a satisfactory effect in the treatment and/or the prevention of susceptive diseases. Although it varies dependently on the types of susceptive diseases and their symptoms, the agent can be orally administered to patients or parenterally administered to patients' intradermal tissues, subcutaneous tissues, muscles, and veins at a dose in the range of about 0.1-50 mg/shot, preferably, about one µg/shot to one mg/shot, 1-4 times/day or 1-5 times/week, for one day to one year.

The agent according to the present description can be also used in so called "antitumor immunotherapy" using interleukin 2. Generally, the antitumor immunotherapy is roughly classified into (i) a method for directly administering interleukin 2 to the body of patients with malignant tumors, and (ii) a method for introducing antitumor cells activated *in vitro* by interleukin 2 (adoptive immunotherapy). The immunotherapeutic effect can be significantly enhanced when administered to along with the polypeptide. In the method (i), the polypeptide is administered to patients in an amount of about 0.1 µg/shot/adult to one mg/shot/adult at 1-10 times simultaneously or before the administration of interleukin 2. The dose of interleukin 2 is generally set to a dose in the range of about 10,000 to 1,000,000 units/shot/adult, though it varies dependently on the types of malignant tumors, patients' symptoms, and the polypeptide dose. While in the method (ii), mononuclear cells and lymphocytes, collected from patients with malignant tumors, are cultured in the presence of interleukin 2 and about one ng to one mg of the polypeptide per 1x10⁶ cells of these blood cells. After culturing for a prescribed period of time, NK cells and LAK cells were collected from the culture, and introduced into the patients' body. Diseases which can be treated by the present antitumor immunotherapy are, for example, solid malignant tumors such as colonic cancer, rectal cancer, gastric cancer, thyroid carcinoma, cancer of the tongue, bladder carcinoma, choriocarcinoma, hepatoma, prostatic cancer, carcinoma uteri, laryngeal, lung cancer, breast cancer, malignant melanoma, Kaposi's sarcoma, cerebral tumor, neuroblastoma, tumor of the ovary, testicular tumor, osteosarcoma, cancer of the pancreas, renal cancer, hypernephroma, hemangioendothelioma, and blood cell malignant tumors such as leukemia and malignant lymphoma.

The following Examples explain the present invention, and the recombinant DNA technology used therein are in themselves conventionally known in the art: For example, such a technology is disclosed by J. Sumbrook et al. in "Molecular Cloning, A Laboratory Manual", 2nd edition (1989), published by Cold Spring Harbor Laboratory Press, New York, USA, and by Masami MURAMATSU in "Laboratory Manual for Genetic Engineering" (1988), published by Maruzen Co., Ltd., Tokyo, Japan.

### Example A-1

### Preparation of purified polypeptide

To 600 female CD-1 mice, 8-week-old, was intraperitonealy injected one mg/mouse of dead *Corynebacterium parvum* (ATCC 11827) which had been preheated at 60°C for one hour, and the mice were fed in usual manner for 7 days and intravenously injected with one µg/mouse of a purified lipopolysaccharide derived from *Escherichia coli*. On 1-2 hours after the intravenous injection, the mice were sacrificed to collect their blood, followed by removing their livers, disrupting the livers with a homogenizer in 8-fold volumes of 50 mM phosphate buffer (pH 7.3), and extracting the resultant suspension. The resultant extract was centrifuged at about 8,000 rpm for 20 min, and an about 9 L of the supernatant was admixed with a saturated ammonium sulfate in 50 mM phosphate buffer (pH 7.3) to give a saturation degree of 45 w/v %. The resultant solution was allowed to stand at 4°C for 18 hours and centrifuged at about 8,000 rpm for 30 min to obtain an about 19 L supernatant containing the present polypeptide.

The supernatant was fed to a column packed with about 4.6 L of "PHENYL SEPHAROSE", a product of Pharmacia LKB Biotechnology AB, Uppsala Sweden, which had been equilibrated with 50 mM phosphate buffer (pH 7.3) containing one M ammonium sulfate, and the column was washed with a fresh preparation of the same buffer, and fed at an SV (space velocity) 0.57 with a linear gradient buffer ranging from 1 M to 0.2 M ammonium sulfate in 50 mM phosphate buffer (pH 7.3). Fractions containing the present polypeptide eluted at 0.8 M ammonium sulfate were collected and pooled into an about 4.8 L solution which was then concentrated with a membrane filter, dialyzed against 20 mM phosphate buffer (pH 6.5) at 4°C for 18 hours, and fed to a column packed with about 250 ml of "DEAE-SEPHAROSE", a product of Pharmacia LKB Biotechnology AB, Uppsala, Sweden. The column was washed with a fresh preparation of the same buffer and fed at an SV 1.2 with a linear gradient buffer ranging from 0 M to 0.2 M sodium chloride in 20 mM phosphate buffer (pH 6.5) to elute and collect about 260 ml fractions containing the present polypeptide eluted at a concentration of about 0.13 M sodium chloride.

Fractions containing the present polypeptide were collected, pooled, concentrated and dialyzed against 25 mM Bis-Tris buffer (pH 7.1) at 4°C for 18 hours. The dialyzed solution was applied to a column packed with about 24 ml of "MONO-P", a product of Pharmacia LKB Biotechnology AB, Uppsala, Sweden, and eluted with 10 v/v % polybuffer 74 (pH 4.0) while decreasing the pH from 7 to 4 to obtain an about 23 ml eluate containing the present polypeptide. The eluate was concentrated, fed to a column packed with "SUPER-DEX 75", a product of Pharmacia LKB Biotechnology AB, Uppsala, Sweden, which had been equilibrated with a mixture solution (pH 7.2) containing 7 mM disodium hydrogen phosphate, 3 mM sodium dihydrogen phosphate, and 139 mM sodium chloride, and subjected to gel filtration chromatography to elute fractions, containing the present polypeptide at around 19,000 daltons, with a fresh preparation of the same solution. The fractions were pooled and concentrated for use in Example A-2. The yield of the present polypeptide was about 0.6 µg/mouse.

### Example A-2

### Partial amino acid sequence of polypeptide

A portion of an aqueous solution containing the purified polypeptide in Example A-1 was concentrated up to a volume of about 50 µl which was then admixed with 25 µl of a solution containing 3 w/v % SDS, 60 v/v % glycerol, and 60 mg/ml dithiothreitol. The resultant mixture was incubated at 50°C for 30 min, positioned on 15 w/v % polyacrylamide gel, and electrophoresed in usual manner. The resultant gel was stained by soaking it in a mixture solution of 10 v/v % aqueous acetic acid solution and 50 v/v % aqueous methanol containing 0.1 w/v % coomassie brilliant blue R 250, destained by repeatedly washing the gel with a mixture solution of 12 v/v % aqueous methanol and 7 v/v % aqueous acetic acid solution, and washed by soaking it in distilled water for 18 hours. A portion of the gel, which was stained with the coomassie brilliant blue and contained the present polypeptide, was cut out of the gel, and lyophilized.

The lyophilized gel was soaked in 0.6 ml solution consisting of 100 mM sodium hydrogen carbonate containing 2 µg/ml "TPCK TRYPSIN", 0.5 mM calcium chloride, and 0.02 v/v % aqueous Tween 20 solution, followed by the incubation at 37°C for 18 hours to trypsinize the protein. The resultant was centrifuged to obtain a supernatant, while the resultant precipitate was soaked in one ml of one v/v % aqueous trifluoroacetate containing 0.001 v/v % Tween 20, shook for 4 hours at ambient temperature, and centrifuged to obtain a supernatant. The newly formed precipitate was successively treated similarly as above with 70 v/v aqueous trifluoroacetate containing 0.001 v/v Tween 20 and with 50 v/v % aqueous acetonitrile to obtain a supernatant. The resultant supernatant and the already obtained supernatant in the above were pooled and concentrated up to give 250 µl which was then centrifugally filtered.

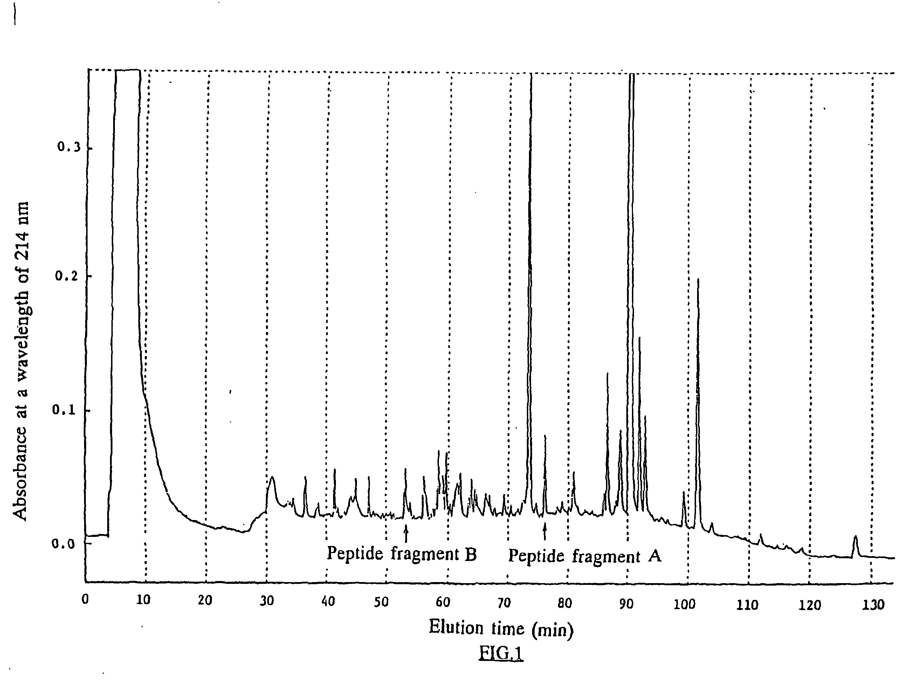

The resultant aqueous solution containing peptide fragments was fed to "HPLC ODS-120T", a column for HPLC commercialized by Tosoh Corporation, Tokyo, Japan, which had been previously equilibrated with 0.1 v/v aqueous trifluoroacetate, and the column was washed with 0.1 v/v % aqueous trifluoro acetate, and fed with 0.1 v/v % trifluoro acetate at a flow rate of 0.5 ml/min while the concentration of aqueous acetonitrile was increasing from 0 v/v % to 70 v/v % and the concentration of peptide in the eluate was monitoring by a spectrophotometer at wave lengths of 214 nm and 280 nm. Fractions eluted about 75 min and about 55 min after initiating the elution were respectively collected (hereinafter named "peptide fragment A" and "peptide fragment B"). The elution pattern was in FIG.1.

The peptide fragments A and B were analyzed on "MODEL 473 A", a protein sequencer commercialized by Perkin-Elmer Corp., Instrument Div., Norwalk, USA, and revealing that they have the amino acid sequences in SEQ ID NOs:4 and 5.

### Example A-3

### Base sequence of DNA encoding protein and

### amino acid sequence of polypeptide

### Example A-3-1

### Preparation of whole RNA

Three g of wet mouse liver cells, similarly prepared by the method in Example A-1, was weighed, soaked in 20 ml of a mixture solution containing 6 M guanidine isothiocyanate, 10 mM sodium citrate, and 0.5 w/v SDS, and disrupted with a homogenizer. Thirty-five-ml centrifugation tubes were injected with 25 ml of 0.1 M EDTA (pH 7.5) containing 5.7 M cesium chloride, and 10 ml of the homogenized cell suspension was overlaid on the upper part of the solutions in the tubes, followed by centrifuging the tubes at 25,000 rpm for 20 hours to collect RNA fractions. The fractions were pooled, distributed into 15-ml centrifugation tubes, and mixed with equal volumes of a mixture solution of chloroform and isobutanol (= 4:1 by volume). The tubes were vibrated for 5 min and centrifuged at 4°C and at 10,000 rpm for 10 min, and the formed water layers were collected, pooled, mixed with 2.5-fold volumes of ethanol, and allowed to stand at -20°C for 2 hours to precipitate the whole RNAs. The precipitate was collected, pooled, washed with 75 v/v % aqueous ethanol, and dissolved in 0.5 ml of sterilized distilled water for use in Example A-3-2. The yield of the RNAs was about 4 mg, d.s.b.

### Example A-3-2

### Preparation of DNA fragments partially encoding polypeptide

One µg of the whole RNAs in Example A-3-1 was mixed with 4 µl of 25 mM magnesium chloride, 2 µl of a solution consisting of 10xPCR buffer, 100 mM Tris-HCl buffer (pH 8.3) and 500 mM potassium chloride, 8 µl of one mM dNTP mix, one µl of a solution containing one unit/pl RNase inhibitor, one µl of a solution containing 2.5 units/µl reverse transcriptase, and one µl of 2.5 µM random hexamer, and further mixed with water to give a total volume of 20 µl. The mixture solution was placed in 0.5 ml reaction tubes, and, in usual manner, successively incubated at 25°C for 10 min, at 42°C for 30 min, at 99°C for 5 min, and at 5°C for 5 min to effect the reverse transcriptase reaction, followed by recovering an aqueous solution containing the first strand cDNA.

To 20 µl of the aqueous solution were added 4 µl of 25 mM magnesium chloride, 8 µl of 10xPCR buffer, 0.5 µl of a solution containing 2.5 units/µl of AmpliTaq DNA polymerase commercialized by Perkin-Elmer Corp., Instrument Div., Norwalk, USA, and one pmole each of primers 1 and 2 as a sense primer or an anti-sense primer. The mixture solution was volumed up to 100 µl with sterilized distilled water, and, in usual manner, successively incubated at 94°C for one min, at 45°C for 2 min, at 72°C for 3 min in a cyclic manner for 40 cycles to amplify a DNA fragment, which partially encodes the present polypeptide, by using the first strand cDNA as a template. The primers 1 and 2 were oligonucleotides, which were chemically synthesized based on the amino acid sequences of Pro-Glu-Asn-Ile-Asp-Asp-Ile and Phe-Glu-Asp-Met-Thr-Asp-Ile in SEQ ID NOs:4 and 5, had the base sequences of 5'-ATRTCRTCDATRTTYTCNGG-3' and 5'-TTYGARGAYATGACNGAYAT-3'.

A portion of the resultant PCR product was fractionated on electrophoresis in 2 w/v % agarose gel, transferred on a nylon film, fixed with 0.4 N sodium hydroxide, washed with 2xSSC, air-dried, soaked in a prehybridization solution containing 5xSSPE, 5xDenhard's solution, 0.5 w/v % SDS and 100 µg/ml of denatured salmon sperm DNA, and incubated at 65°C for 3 hours. An oligonucleotide as a probe 1 having a base sequence of 5'-TTYGARGARATGGAYCC-3' was synthesized based on the amino acid sequence of Phe-Glu-Glu-Met-Asp-Pro in SEQ ID NO:4, and labeled with [γ-³²P]ATP and T4 polynucleotide kinase.

The nylon film was soaked in a solution containing one pmole of the probe 1, 5xSSPE, 5xDenhardt's solution, 0.5 w/v % SDS, and 100 µg/ml of a denatured salmon sperm DNA, and incubated at 45°C for 24 hours to effect hybridization. The resultant nylon film was washed with 6xSSC and autoradiographed in usual manner and revealing that the PCR product contained the objective DNA fragment.

The remaining PCR product was mixed with 50 ng of "pT7 BLUE T", a plasmid vector commercialized by Takara Shuzo Co., Ltd., Tokyo, Japan, an adequate amount of T4 ligase, and further mixed with 100 mM ATP up to give a concentration of one mM, followed by the incubation at 16°C for 18 hours to insert the DNA fragment into the plasmid vector. The recombinant DNA thus obtained was introduced into *Escherichia coli* NoVa Blue strain, a microorganism of the species *Escherichia coli* commercialized by Pharmacia LKB Biotechnology AB, Uppsala, Sweden, to obtain a transformant which was then inoculated into a medium plate containing 10 g/l bactotryptone, 2.5 g/l sodium chloride, 15 g/l bacto-agar, 100 mg/l ampicillin, 40 mg/l X-Gal and 23.8 mg/l isopropyl-β-D-thiogalacto-pyranoside (hereinafter abbreviated as "IPTG"), and incubated at 37°C for 24 hours to form colonies. A nylon film was in usual manner overlaid on a medium plate and allowed to stand for about 30 seconds to attach the colonies thereunto. The nylon film was then detached from the plate and soaked for 7 min in a solution containing 0.5 N sodium hydroxide and 1.5 M sodium chloride to effect cell lysis. Thereafter, the nylon film was further soaked for 3 min in 0.5 M Tris-HCl buffer (pH 7.2) containing 1.5 M sodium chloride, washed with 2xSSC, soaked in 0.4 N sodium hydroxide for 20 min to fix the DNA, washed with 5xSSC, air-dried, soaked in a prehybridization solution containing 5xSSPE, 5xDenhardt's solution, 0.5 w/v % SDS, and 100 µg/ml denatured salmon sperm DNA, and incubated at 65°C for 3 hours. The colonies formed on the nylon film were in usual manner hybridized with the probe 1, washed with 6xSSC, and autoradiographed similarly as above, followed by selecting transformants which strongly hybridized with the probe 1.

The transformants were inoculated in L-broth (pH 7.2) containing 100 µg/ml ampicillin and incubated at 37°C for 18 hours, followed by collecting cells from the culture and collecting recombinant DNA by conventional alkali-SDS method. The analysis of the dideoxy method revealed that the recombinant DNA contained a DNA fragment which consists of base sequences corresponding to the bases positioning from 85 to 281 in SEQ ID NO:3.

### Example A-3-3

### Preparation of mRNA

0.05 ml of an aqueous solution containing the whole RNAs in Example A-3-1 was placed in a test tube, admixed with 0.5 ml of 10 mM Tris-HCl buffer (pH 7.5) containing one mM EDTA and 0.1 w/v % SDS, and volumed up to one ml with sterilized distilled water. To the mixture was added one ml "OLIGOTEX-dT30 SUPER", an oligo-d(T)₃₀ latex commercialized by Nippon Roche K.K., Tokyo, Japan, followed by the incubation at 65°C for 5 min to denature the RNAs and the cooling for 3 min in an ice-chilled bath. The resultant mixture was admixed with 0.2 ml of 5 M sodium chloride, incubated at 37°C for 10 min, and centrifuged at 10,000 rpm at 25°C for 10 min. The precipitate in the form of a pellet was suspended in 0.5 ml sterilized distilled water, and incubated at 65°C for 5 min to extract mRNA from the oligo-d(T)₃₀ latex. The yield of the mRNA was about 5 µg.

### Example A-3-4

### Preparation of cDNA library

cDNA Library was prepared from the mRNA in Example A-3-3 by using "cDNA SYNTHESIZING SYSTEM PLUS", a cDNA cloning kit commercialized by Amersham Corp., Div., Amersham International, Arlington Heights, USA. The procedures were as follows: To 1.5-ml reaction tube were successively added 4 µl of a solution for synthesizing the first strand cDNA, one µl sodium pyrophosphate solution, one µl of a solution of human placenta ribonuclease inhibitor, 2 µl deoxynucloside triphosphate mix, and one µl oligo-d(T)₁₆ primer. The resultant mixture was mixed with 2 µl of mRNA in Example A-3-3, volumed up to 19 µl with sterilized distilled water, mixed with one µl of a solution containing 20 units of reverse transcriptase, and incubated at 42°C for 40 min to obtain a reaction mixture containing the first strand cDNA.

The mixture thus obtained was mixed with 37.5 µl of a solution for synthesizing the second strand cDNA, 0.8 units of ribonuclease H derived from *Escherichia coli,* 23 units of DNA polymerase, and volumed up to 100 µl with sterilized distilled water. The resultant mixture was successively incubated at 12°C for 60 min and at 22°C for 60 min, mixed with 2 units of T4 DNA polymerase, and incubated at 37°C for 10 min to obtain a reaction mixture containing the second strand cDNA. To the reaction mixture was added 4 µl of 0.25 M EDTA (pH 8.0) to suspend the reaction, and the resultant mixture was in usual manner extracted with phenol and chloroform and treated with ethanol to precipitate the objective cDNA, followed by recovering the precipitate.

To the cDNA thus obtained were added 2 µl of L/K buffer, 250 pmole *Eco* RI adaptor, and 2.5 units of T4 DNA ligase in this order, and the resultant solution was volumed up to 20 µl with sterilized distilled water, and incubated at 15°C for 16 hours to ligate the *Eco* RI adaptor to the both ends of the cDNA. The reaction mixture was mixed with 2 µl of 0.25 M EDTA to inactivate the remaining enzyme, and subjected to molecular sieve chromatography to remove intact *Eco* RI adaptor. To the resultant were added 40 µl of L/K buffer, 80 units of T4 polynucleotide kinase, and the mixture was volumed up to 400 µl with sterilized distilled water, followed by the incubation at 37°C for 30 min to methylate the *Eco* RI cleavage sites. The resultant mixture was extracted with phenol and chloroform and treated with ethanol to precipitate the objective DNA, followed by recovering the DNA. To the DNA were added 1.5 µl of L/K buffer containing an adequate amount of λgt 10 arms, and 2.5 units of T4 DNA ligase, and the resultant solution was volumed up to 15 µl with sterilized distilled water, incubated at 15°C for 16 hours to effect ligation, and subjected to conventional *in vitro* packaging method to obtain a phage containing a recombinant λDNA.

### Example A-3-5

### Cloning of recombinant DNA

A seed culture of *Escherichia coli* NM514 strain was in usual manner infected with the phage in Example A-3-4, and the infected cells were inoculated in an agar plate (pH 7.0) containing 10 g/l bacto-tryptone, 5 g/l bacto-yeast extract, 10 g/l sodium chloride and 15 g/l bacto-agar, and incubated at 37°C for 16 hours to form plaques. The agar plate was covered with a nylon film and allowed to stand for about 30 seconds to attach the plaques thereunto. The nylon film was detached from the plate, and successively soaked in an aqueous solution containing 0.5 M sodium hydroxide and 1.5 M sodium chloride for 7 min and in 0.5 M Tris-HCl buffer (pH 7.0) containing 1.5 M sodium chloride for 3 min. The nylon film was washed with 2xSSC, air-dried, soaked in 0.4 N sodium hydroxide for 20 min, washed with 5xSSC, air-dried, soaked in a solution containing 5xSSPE, 5xDenhardt's solution, 0.5 w/v % SDS, and 100 µg/ml denatured salmon sperm DNA, and incubated at 65°C for 3 hours. Thereafter, the resultant nylon film was incubated in a solution containing an adequate amount of DNA fragment as the probe 2 obtained in Example A-3-2 and labeled with ³²P by "READY PRIME DNA LABELLING SYSTEM", a DNA labeling kit commercialized by Amersham Corp., Div., Amersham International, Arlington Heights, USA, 5xSSPE, 5xDenhardt's solution, 0.5 w/v % SDS, and 100 µg/ml of denatured salmon sperm DNA, and the mixture was incubated at 60°C for 20 hours to effect hybridization. The resultant was subjected to radioautography similarly as above to select phage DNA clones which strongly hybridized with the probe 2.

With conventional techniques, the clones were amplified in *Escherichia coli*, followed by extracting a recombinant DNA from the cells. The recombinant DNA was cleaved with *Eco* RI, a restriction enzyme. Plasmid vector pUC19 (ATCC 37254) was cleaved with the same restriction enzyme, and the resultant cleaved DNA fragments and plasmid fragments were ligated with DNA ligase to obtain a recombinant DNA which was then introduced into *Escherichia coli* JM109 strain (ATCC 53323) by conventional competent cell method to obtain a transformant.

### Example A-3-6

### Determination of base sequence of DNA

### and amino acid sequence of polypeptide

The transformant in Example A-3-5 was inoculated into L-broth (pH 7.2) and cultured at 37°C for 18 hours under shaking conditions. The resultant proliferated cells were collected and treated with conventional alkali-SDS method to obtain a recombinant DNA containing the DNA according to the present invention. The analysis on an automatic sequencer using a fluorophotometer revealed that the recombinant DNA contains the base sequence in SEQ ID NO:3. The decoding of the base sequence indicated that it encodes the amino acid sequence in SEQ ID NO:3. The amino acid sequence contains the partial amino acid sequences in SEQ ID NOs:4 and 5 corresponding to the amino acids positioning from 79 to 103 and from 26 to 43 in SEQ ID NO:3, and this means that in mice the polypeptide having the amino acid sequence in SEQ ID NO:3 is also encoded by the DNA in SEQ ID NO:3 where the symbol "Xaa" means "methionine" or "threonine".

In the following Examples A-4 to A-7, a cDNA, which encodes another polypeptide that induces the IFN-γ production by immunocompetent cells, is prepared from human liver mRNA by using as a probe a DNA fragment of the base sequence in SEQ ID NO:3. The cDNA was analyzed for base sequence and decoded to determine the amino acid sequence of the polypeptide. The cDNA was allowed to express in *Escherichia coli*, followed by studying the feature and property of the formed polypeptide.

### Example A-4

### Base sequence of DNA encoding polypeptide and

### amino acid sequence of polypeptide

### Example A-4-1

### Preparation of cDNA library

cDNA Library was prepared from a human liver RNA supplemented with "POLY A", a product commercialized by Clonatec-BIOSOFT, Paris Cedex, France, by using "cDNA SYNTHESIZING SYSTEM PLUS", a cDNA cloning kit commercialized by Amersham Corp., Div., Amersham International, Arlington Heights, USA. The procedures were as follows: To 1.5-ml reaction tube were successively added 10 µl of a solution for synthesizing the first strand cDNA, 2.5 µl of one mM sodium pyrophosphate, 2.5 µl of a solution containing one µg/l of a human placenta ribonuclease inhibitor, 5 µl of a solution containing one µg/l of a deoxynucleotide triphosphate mix, 2.5 µl of a solution containing one µg/l oligo-dT primer, 5 µl of a human liver RNA supplemented with poly(A), and volumed up to 45 µl with sterilized distilled water. Thereafter, the resultant mixture was mixed with 5 µl of a solution containing 100 units of a reverse transcriptase, and incubated at 42°C for 40 min to obtain a reaction mixture containing the first strand cDNA.

To the reaction mixture was added 93.5 µl of a solution for synthesizing the second strand cDNA, 4 units of ribonuclease H derived from *Escherichia coli*, 115 units of DNA polymerase, and volumed up to 250 µl with sterilized distilled water. The resultant mixture was successively incubated at 12°C for 60 min, at 22°C for 60 min, and at 70°C for 10 min, mixed with 10 units of T4 polymerase, and further incubated at 37°C for 10 min. To the reaction mixture was added 10 µl of 0.25 M EDTA (pH 8.0) to suspend the reaction, and the resultant mixture was in usual manner extracted with phenol and chloroform, and treated with ethanol to precipitate the objective second strand cDNA, followed by recovering the precipitate.

To the second strand cDNA thus obtained were added 2 µl L/K buffer (pH 8.0), 250 pmole *Eco* RI adaptor, and 2.5 units of T4 DNA ligase, and the resultant solution was volumed up to 20 µl with sterilized distilled water, and incubated at 15°C for 16 hours to ligate the *Eco* RI adaptor to the both ends of the cDNA. The resultant mixture was then mixed with 2 µl of 0.25 M EDTA to suspend the reaction, and subjected to molecular sieve chromatography to remove intact *Eco* RI adaptor. To the resultant were added 40 µl of L/K buffer (pH 8.0) and 80 units of T4 polynucleotide kinase, and the mixture was volumed up to 400 µl with sterilized distilled water, followed by the incubation at 37°C for 30 min to methylate the *Eco* RI cleavage sites. The resultant mixture was extracted with phenol and chloroform and treated with ethanol to precipitate the objective cDNA, followed by recovering the cDNA. To the cDNA were added 1.5 µl of L/K buffer (pH 8.0) containing an adequate amount of λgt 10 arms, and 2.5 units of T4 DNA ligase, and the resultant solution was volumed up to 15 µl with sterilized distilled water, incubated at 15°C for 16 hours to effect ligation, and subjected to conventional *in vitro* packaging method to obtain a phage containing a recombinant λDNA.

### Example A-4-2

### Cloning of recombinant DNA

A seed culture of *Escherichia coli* NM514 strain was in usual manner infected with the phage in Example A-4-1, and the infected cells were inoculated in an agar plate (pH 7.0) containing 10 g/l bacto-trypton, 5 g/l bacto-yeast extract, 10 g/l sodium chloride, and 15 g/l bacto-agar, and incubated at 37°C for 16 hours to form plaques. According to conventional method, the agar plate was covered with a nylon film and allowed to stand for about 30 seconds to attach the plaques thereunto. Thereafter, the nylon film was detached from the plate, and successively soaked in an aqueous solution containing 0.5 N sodium hydroxide and 1.5 M sodium chloride for 7 min and in 0.5 M Tris-HCl buffer (pH 7.0) containing 1.5 M sodium chloride for 3 min. The nylon film was washed with 2xSSC, air-dried, soaked in 0.4 N sodium hydroxide for 20 min, washed with 5xSSC, air-dried, soaked in a solution containing 5xSSPE, 5xDenhardt's solution, 0.5 w/v % SDS and denatured salmon sperm DNA, and incubated at 65°C for 3 hours. To clone the objective recombinant DNA, a DNA fragment having the base sequence in SEQ ID NO:3 was labeled with ³²P by "READY PRIME DNA LABELLING SYSTEM", a DNA labeling kit commercialized by Amersham Corp., Div., Amersham International, Arlington Heights, USA, to obtain probe 3. The procedures were as follows: Place in 1.5-ml reaction tube 25 ng of a DNA fragment prepared by the method in Example A-3-5, volumed up to 45 µl of sterilized distilled water, incubated at 95°C for 3 min, and transferred to another reaction tube. Five µl of [α-³²P]dCTP solution was added to the reaction tube, and labeled by incubating it at 37°C for 30 min. Thereafter, the resultant product containing the labeled DNA fragment was subjected to conventional molecular sieve chromatography to remove intact [α-³²P].

The above nylon film was soaked in a mixture solution containing 5xSSPE, 5xDenhardt's solution, 0.5 w/v % SDS, and 100 µg/ml of a denatured salmon sperm DNA, and the mixture was incubated at 60°C for 20 hours to effect hybridization, and further incubated at ambient temperature in 6xSSC for 20 min and in 2xSSC for 20 min. The resultant was washed and subjected to autoradiography similarly as above to select phage DNA clones which strongly hybridized with the probe 3. With conventional techniques, the DNA clones were amplified in *Escherichia coli*, followed by the extraction of a recombinant DNA from the cells. The recombinant DNA was cleaved with *Eco* RI, a restriction enzyme. Plasmid vector pUC19 (ATCC 37254) was cleaved with the same restriction enzyme, and the cleaved DNA fragments and plasmid fragments were ligated with DNA ligase to obtain a recombinant DNA which was then introduced into *Escherichia coli* JM109 strain (ATCC 53323) by conventional competent cell method to obtain a transformant containing the present DNA.

### Example A-4-3

### Determination of base sequence and amino acid sequence

### of polypeptide

The transformant in Example A-4-2 was inoculated into L-broth (pH 7.2) containing 50 µg/ml of ampicillin, and cultured at 37°C for 18 hours under shaking conditions. The proliferated cells were collected by centrifugation and treated with conventional alkali-SDS method to extract a recombinant DNA. The analysis of the base sequence on an automatic sequencer using a fluorophotometer revealed that the recombinant DNA contains the base sequence in SEQ ID NO:6. The amino acid sequence estimable from the base sequence is also shown in SEQ ID NO:6, and this indicates that the present polypeptide has an amino acid sequence, for example, the one in SEQ ID NO:1, and that the polypeptide is encoded by the DNA of the base sequence in SEQ ID NO:2. In SEQ ID NO:6, the amino acid as shown by "Xaa" means "isoleucine" or "threonine".

### Example A-5

### Preparation of replicable recombinant DNA and transformant

To a 0.5-ml reaction tube were added 8 µl of 25 mM magnesium chloride, 10 µl of 10xPCR buffer, 8 µl of one mM dNTP mix, 0.5 µl of a solution containing 2.5 units/µl AmpliTaq DNA polymerase, and one ng of the recombinant DNA in Example A-4-2. The resultant mixture was mixed with adequate amounts of 2 oligonucleotides, as a sequence primer or anti-sense primer, having base sequences represented by 5'-CGAGGGATCCTACTTTGGCAAGC TTG-3' and 5'-CAAGGAATTCCTAGTCTTCGTTTTG-3' which had been chemically synthesized based on the base sequences near to the N- and C-termini in SEQ ID NO:1, and volumed up to 100 µl with sterilized distilled water. The resultant mixture was in usual manner successively incubated at 94°C for one min, at 60°C for 2 min, and at 72°C for 3 min, and this incubation cycle was repeated for 40 times to obtain a PCR product which was then cleaved with *Bam* HI and *Eco* RI as restriction enzymes to obtain a *Bam* HI-*Eco* RI DNA fragment. The resultant *Bam* HI-*Eco* RI DNA fragment was mixed with an adequate amount of sterilized distilled water. The solution was mixed with 10 ng "pGEX-2T", a plasmid vector commercialized by Pharmacia LKB Biotechnology AB, Uppsala, Sweden, which had been previously cleaved with *Bam* HI and *Eco* RI as a restriction enzyme, 10 µl of 10xligation buffer, and an adequate amount of 10 mM ATP to give a final concentration of one mM, followed by the incubation at 16°C for 18 hours to obtain the replicable recombinant DNA pHIGIF.

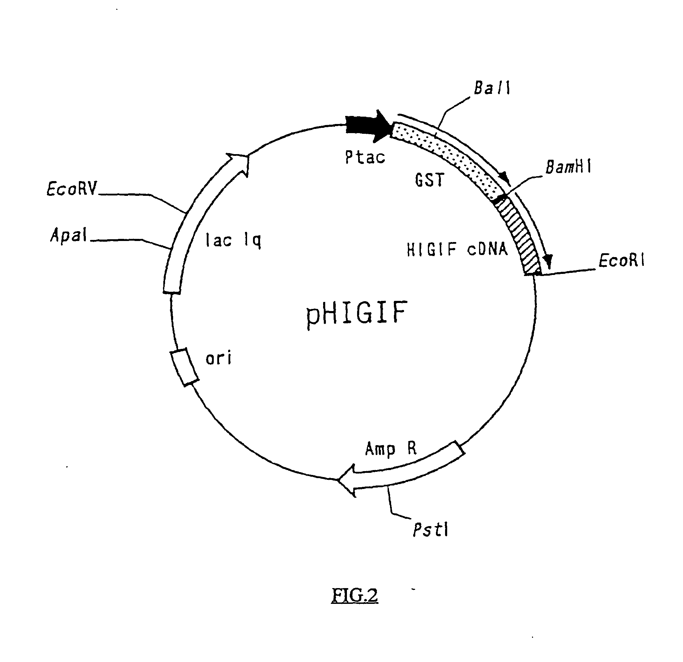

The recombinant DNA pHIGIF was introduced into *Escherichia coli* DH5a strain commercialized by Toyobo Co., Ltd., Tokyo, Japan, and the resultant transformant "HIGIF" was inoculated into L-broth (pH 7.2) containing 50 µg/ml ampicillin, and incubated at 37°C for 18 hours under shaking conditions. The resultant culture was centrifuged to obtain the proliferated transformants which were then subjected to conventional alkali-SDS method to extract the recombinant DNA pHIGIF. The analysis of the recombinant pHIGIF on the dideoxy method revealed that as shown in FIG.2 "HIGIF cDNA" or the cDNA in SEQ ID NO:2 ligated to the sites in the downstream of genes for Tac promotor and glutathione S-transferase.

### Example A-6

### Production of polypeptide from transformant

The transformant HIGIF in Example A-5 was inoculated into T-broth (pH 7.2) containing 50 µg/ml of ampicillin, and incubated at 37°C for 18 hours under shaking conditions to obtain a seed culture. Eighteen L aliquots of a fresh preparation of T-broth (pH 7.2) were placed in 30-L jar fermenters, inoculated with one v/v % of the seed culture, and cultured at 37°C under aeration-agitation conditions. During the cultivation, the culture was sampled and monitored for absorbance at a wave length of 650 nm, and, when the absorbance reached to about 1.5, IPTG was added to the culture up to give 0.1 mM. Thereafter, the culture was further incubated for another 5 hours and centrifuged to separate cells from the culture. The cells were suspended in a mixture solution (pH 7.2) containing 139 mM sodium chloride, 7 mM disodium hydrogen phosphate, and 3 mM sodium dihydrogen phosphate, treated in usual manner with ultrasonic, and centrifuged to obtain a supernatant.

The supernatant was fed to a column packed with "GLUTATHIONE SEPHAROSE 4B", a product of Pharmacia LKB Biotechnology AB, Uppsala, Sweden, which had been previously equilibrated with a mixture solution (pH 7.2) containing 139 mM sodium chloride, 7 mM disodium hydrogen phosphate and 3 mM sodium dihydrogen phosphate. The column was washed with a fresh preparation of the same mixture solution, and 100 U of thrombin was added to one ml of the gel in the column to effect enzymatic cleavage reaction while allowing the column to stand at ambient temperature for 16 hours. The column was fed with a fresh preparation of the same mixture solution to elute the reaction product, and the eluate was fed to a column packed with "SUPERDEX 75", a product of Pharmacia LKB Biotechnology AB, Uppsala, Sweden, followed by collecting fractions corresponding near to 18,500 daltons. The fractions were pooled, concentrated and lyophilized to obtain a solid product containing the present polypeptide in a yield of about 80 µg per one L of the culture.

### Example A-7

### Physicochemical property of polypeptide

### Example A-7-1

### Molecular weight

In accordance with the method reported by U. K. Laemmli in Nature, Vol.227, pp.680-685 (1970), the purified polypeptide prepared by the method in Example A-6 was electrophoresed in a sodium dodecyl sulfate (SDS) polyacrylamide gel free of reducing agent to mainly show a single protein band with an IFN-γ inducibility at a position corresponding to about 18,500±3,000 daltons. The marker proteins used in this experiment were calf serum albumin (MW=67,000 daltons), ovalbumin (MW=45,000 daltons), soy bean trypsin inhibitor (MW=20,100 daltons), and α-lactalbumin (MW=14,400 daltons).

### Example A-7-2

### Isoelectric point

The purified polypeptide in Example A-6 was chromatofocused to show an isoelectric point of about 4.9±1.0. Example A-7-3

### Amino acid sequence containing the N-terminus

The purified polypeptide in Example A-6 was analyzed on "MODEL 473 A", a protein sequencer commercialized by Perkin-Elmer Corp., Instrument Div., Norwalk, USA, and revealing that it has the structure wherein a peptide, "Gly-Ser-", coupled to the tyrosine residue in the N-terminal amino acid sequence in SEQ ID NO:7 by the addition of glutathione S-transferase and by the cleavage with thrombin.

### Example A-7-4(a)

### Biological activity

From female C3H/HeJ mice, 8-week-old, were extracted their spleens which were then suspended in serum-free RPMI 1640 medium (pH 7.4), and the resultant cells were washed with a fresh preparation of the same medium, and soaked in Gey solution (pH 8.0) to effect hemolysis. The resultant spleen cells were suspended in RPMI 1640 medium (pH 7.4) supplemented with 10 v/v % calf serum to give a cell density of 1x10⁷ cells/ml. Ten ml aliquots of the cell suspension were distributed into plastic petri dishes, 9 cm in diameter, and incubated at 37°C for one hour in a 5 v/v % CO₂ incubator. Only cells floating in the resultant cultures were collected and washed with RPMI 1640 medium (pH 7.4) supplemented with 10 v/v % calf serum for use in the following test for IFN-γ induction.

Mouse spleen cells were suspended in RPMI 1640 medium (pH 7.4) supplemented with 10 v/v % calf serum to give a cell density of 1x10⁷ cells/ml, and 0.15 ml aliquots of which were injected into 96-well microplates, followed by adding to each well 0.05 ml of a solution of a purified polypeptide diluted with a fresh preparation of the same medium, and incubating the cells with or without the addition of 0.05 ml of 2.5 µg/ml of concanavalin A or 50 units/ml of interleukin 2, and incubating the resultant at 37°C for 24 hours in a 5 v/v % CO₂ incubator. After completion of the culture, the resultant supernatant in each well was sampled by 0.1 ml to assay the activity of the formed IFN-γ with enzyme immunoassay. As a control, a system similar to the above system was provided and similarly treated as above except for not using the purified polypeptide, concanavalin A and interleukin 2. As an IFN-γ standard, a mouse IFN-γ preparation Gg02-901-533, obtained from the National Institutes of Health, USA, was used and the activity was expressed with international units (IU). The results were in Table 1.

**Table 1**

| Sample concentration (µg/ml) | IFN-γ production by mouse spleen cell (IU/ml) | | |
|---|---|---|---|
| | Sample | Sample plus concanavalin A | Sample plus interleukin 2 |
| 10.00 | 12 | 138 | 118 |
| 3.33 | 6 | 88 | 55 |
| 1.11 | 5 | 56 | 16 |
| 0.37 | 5 | 21 | 12 |
| 0.12 | 5 | 12 | 10 |
| 0.04 | 5 | 11 | 7 |
| 0 | 0 | 4 | 1 |

| | | | |
|---|---|---|---|
| Note : In the Table "Sample" means the present polypeptide. | | | |

### Example A-7-4(b)

### Induction of IFN-γ production from human lymphocyte

By using a syringe containing heparin, a healthy donor was collected blood which was then diluted by 2-fold with serum-free RPMI 1640 medium (pH 7.4), and overlaid on ficoll. The resultant was centrifuged at 2,000 rpm for 20 min to obtain lymphocytes which were then washed with RPMI 1640 medium (pH 7.4) supplemented with 10 v/v % calf serum, suspended in a fresh preparation of the same medium to give a cell density of 5x10⁶ cells/ml, and treated similarly as in Example A-7-4(a) except that a human IFN-γ standard, Gg23-901-530, obtained from the National Institutes of Health, USA, was used as an IFN-γ standard. The results were in Table 2.

**Table 2**

| Sample concentration (µg/ml) | IFN-γ production by human lymphocyte (IU/ml) | | |
|---|---|---|---|
| | Sample | Sample plus concanavalin A | Sample plus interleukin 2 |
| 10.00 | 191 | 479 | 1,182 |
| 3.33 | 169 | 576 | 1,419 |
| 1.11 | 168 | 426 | 1,106 |
| 0.37 | 150 | 296 | 739 |
| 0.12 | 74 | 193 | 390 |
| 0.04 | 36 | 137 | 324 |
| 0 | 1 | 11 | 24 |

| | | | |
|---|---|---|---|
| Note : In the Table "Sample" means the present polypeptide. | | | |

The results in Tables 1 and 2 evidence that the present polypeptide has an activity of inducing IFN-γ production by immunocompetent cells of mammals including human and mouse. In the control groups, any significant IFN-γ production was not found, while in the systems with the polypeptide a significant IFN-γ production was observed. This activity of the polypeptide is strongly augmented when used in combination with concanavalin A or interleukin 2 as a cofactor.

### Example A-7-4(c)

### Production of IFN-γ by immunocompetent cell

Fresh blood was collected from healthy volunteers with heparinized syringes, and diluted with serum-free RPMI 1640 medium (pH 7.4) by 2 folds. The diluted blood was overlaid on Ficoll and centrifuged to obtain lymphocytes which were then washed with RPMI 1640 medium (pH 7.4) supplemented with 10 v/v % fetal calf serum, and suspended in a fresh preparation of the same medium to give a cell density of 5x10⁶ cells/ml. The cell suspension was distributed to 96-well microplates in an amount of 0.15 ml/well.

A polypeptide obtained by the method in Example B-1-2 was diluted to give an appropriate concentration with RPMI 1640 medium (pH 7.4) supplemented with 10 v/v % fetal calf serum, and the diluted solution was distributed to the microplates in an amount of 0.05 ml/well, followed by adding to the microplates 0.05 ml/well of a fresh preparation of the same medium supplemented with or without 2.5 µg/ml of concanavalin A or 50 units/ml of a recombinant human interleukin 2, and incubating the microplates at 37°C for 24 hours in an incubator under 5 v/v % CO₂ conditions. After the cultivation, 0.1 ml of a culture supernatant in each well was sampled and assayed for IFN-γ content with conventional enzyme immunoassay. As a control, a system free of the polypeptide was provided, and similarly treated as above. The results were in Table 3. In the Table, the IFN-γ content was calibrated using Gg23-901-530, an International Standard for Interferon, Human (HuIFN-γ), obtained from National Institute of Health, Bethesda, MD, USA, and expressed by international units (IU).

**Table 3**

| Polypeptide concentration (ng/ml) | IFN-γ productivity by lymphocyte (IU/ml) | | |
|---|---|---|---|
| | Polypeptide | Polypeptide plus 0.5 µg/ml of concanavalin A | Polypeptide plus 10 U/ml of interleukin 2 |
| 0 | 0 | 0 | 0 |
| 1.6 | 1±2 | 92±32 | 184±12 |
| 8.0 | 3±1 | 220±21 | 397±31 |
| 40.0 | 6±4 | 380±34 | 526±28 |
| 200.0 | 14±6 | 549±105 | 637±99 |

The results in Table 3 show that lymphocytes as an immunocompetent cell produced IFN-γ when the polypeptide acts on them. As is evident from the results, the combination use of the polypeptide and interleukin 2 or concanavalin A as a cofactor enhanced the IFN-γ production.

### Example A-7-4(d)

### Enhancement of cytotoxicity by NK cell

Fresh blood was collected from health volunteers with heparinized syringes, and diluted by 2 folds with 10 mM phosphate buffer (pH 7.4) containing 140 mM sodium chloride. The blood was overlaid on PERCOLL, and the resultant was centrifuged, and further subjected to PERCOLL gradient centrifugation to obtain a high-density lymphocytes.

The lymphocytes were suspended in RPMI 1640 medium (pH 7.2) containing 10 µg/ml kanamycin, 5x10⁻⁵ M 2-mercaptoethanol, and 10 v/v % fetal calf serum to give a cell density of 1x10⁶ cells/ml, and the suspension was distributed into 12-well microplates in an amount of 0.5 ml/well. A polypeptide obtained by the method in Example B-1-2 was appropriately diluted with a fresh preparation of the same medium, and the diluted solution was distributed to the microplates in an amount of 1.5 ml/well, followed distributing to the microplates 0.5 ml/well of a fresh preparation of the same medium with or without 50 units/ml of a recombinant human interleukin 2, incubating the microplates in an incubator at 37°C for 24 hours under 5 v/v % CO₂ conditions, washing the microplates with 10 mM phosphate buffer (pH 7.4) containing 140 mM sodium chloride to obtain cultured lymphocytes containing NK cells as an effector cell. K-562 cells (ATCC CCL 243), derived from human chronic myelogenous leukemia, as an NK cell-susceptive target cell which was labelled in usual manner with ⁵¹Cr, were distributed to 96-well microplates to give 1x10⁴ cells/well, and the effector cells were added to each well in the ratio ((effector cells):(target cells)) of 2.5:1, 5:1 or 10:1, and incubated in an incubator at 37°C for 4 hours under 5 v/v % CO₂ conditions. According to conventional method, the radioactivity of each supernatant in each well was measured to count the dead target cells. In each system, the percentage (%) of the dead target cells to the target cells was calculated to determine the cytotoxicity level. The results were in Table 4.

**Table 4**

| Concentration of polypeptide (pM*) | Concentration of interleukin 2 (unit/ml) | Cytotoxicity (%) | | |
|---|---|---|---|---|
| | | (Effector cell):(Target cell) (Effector cell):(Target cell) | | |
| | | 2.5:1 | 5:1 | 10:1 |
| 0 | 0 | 22 | 35 | 65 |
| 0 | 10 | 30 | 48 | 73 |
| 0.5 | 0 | 23 | 36 | 66 |
| 0.5 | 10 | 32 | 50 | 75 |
| 5 | 0 | 25 | 39 | 68 |
| 5 | 10 | 35 | 52 | 78 |
| 50 | 0 | 29 | 47 | 73 |
| 50 | 10 | 41 | 59 | 85 |
| 500 | 0 | 37 | 50 | 83 |
| 500 | 10 | 52 | 70 | 93 |

| | | | | |
|---|---|---|---|---|
| Note : *In the Table, the symbol "pM" means 10⁻¹² M. | | | | |

The results in Table 4 show that the polypeptide has an activity of enhancing the cytotoxicity by NK cells. As is shown in Table 4, the coexistence of interleukin 2 more enhances the cytotoxicity.

### Example A-7-4(e)

### Induction of LAK cell formation

According to conventional manner, ⁵¹Cr-labelled Raji cells (ATCC CCL 86), derived from human Burkitt lymphoma as a target cell non-susceptive to NK cells, were placed in 96-well microplates to give 1x10⁴ cells/well, and cultured for 72 hours. Cultured lymphocytes, containing LAK cells as an effector cell similarly prepared as in Example A-7-4(d), and target cells were added to the microplates in the ratio of 5:1, 10:1 or 20:1, and the microplates were incubated in an incubator at 37°C for 4 hours under 5 v/v % CO₂ conditions. Thereafter, the radioactivity of each supernatant in each well was measured, and the cytotoxicity (%) was calculated similarly as in Example A-7-4(d). The results were in Table 5.

**Table 5**

| Concentration of polypeptide (pM*) | Concentration of interleukin 2 (unit/ml) | Cytotoxicity (%) | | |
|---|---|---|---|---|
| | | (Effector cell):(Target cell) | | |
| | | 5:1 | 10:1 | 20:1 |
| 0 | 0 | 11 | 21 | 34 |
| 0 | 10 | 15 | 28 | 38 |
| 0.5 | 0 | 13 | 22 | 35 |
| 0.5 | 10 | 17 | 31 | 43 |
| 5 | 0 | 15 | 23 | 39 |
| 5 | 10 | 19 | 34 | 48 |
| 50 | 0 | 20 | 25 | 44 |
| 50 | 10 | 23 | 42 | 54 |
| 500 | 0 | 27 | 34 | 57 |
| 500 | 10 | 31 | 54 | 67 |

| | | | | |
|---|---|---|---|---|
| Note : *In the Table, the symbol "pM" means 10⁻¹² M. | | | | |

The results in Table 5 show that the polypeptide has an activity of inducing the formation of LAK cells. As is shown in the results, the coexistence of interleukin 2 more enhances the induction.

### Example A-7-4(f)

### Acute toxicity test

According to conventional manner, a purified polypeptide obtained by the method in Example B-1-2 was percutaneously, perorally or intraperitoneally administered to 8-week-old mice. As a result, the LD₅₀ of the purified polypeptide was about one mg/kg or higher independently of the administration routes. This evidences that the polypeptide can be safely incorporated into pharmaceuticals for administering human.

As is well known that IFN-γs deeply relate to human biophylaxis through the infectious protection against bacteria, growth inhibitory activity for malignant tumors, and immunoregulatory activity. As is described above, the IFN-γs have developed as an agent for human susceptive diseases, and the objective diseases, doses, administration routes, and safeness were substantially studied. As is described in "Cytokines in Cancer Therapy", edited by Frances R. Balkwill, translated by Yoshihiko WATANABE (1991), published by Tokyo-Kagaku-Dojin, Tokyo, Japan, it is reported that almost satisfactory results were obtained when the treatment using killer cells such as NK cells and LAK cells was applied on a variety of human diseases including antitumor immunotherapy. Recently, it is noted that there is a relationship between the therapeutic effect and the induction of killer cells or the enhancement of the cytotoxicity by killer cells using cytokines. For example, T. FUJIOKA reported in "British Journal of Urology", Vol.73, No.1, pp.23-31 (1994) that, in the antitumor immunotherapy using LAK cells and interleukin 2, the interleukin 2 strongly induced the LAK cell formation and exerted a remarkable cancer metastasis-inhibitory activity on human cancers without inducing serious side effects.

Thus, it is revealed that IFN-γs and killer cells deeply relate to the treatment and/or prevention of a variety of human diseases, and greatly contribute to their complete treatment or remission. In these circumstances and as is evident from the results in Examples A-7-4(c) and A-7-4(f), the polypeptide induces the IFN-γ production by immunocompetent cells, and enhances the cytotoxicity by NK cells or induces the formation of LAK cells without causing serious side effects. These facts show that the present susceptive diseases can be repeatedly administered to human without inducing serious side effects, and exerts a satisfactory effect in the treatment and/or the prevention of diseases closely relating to IFN-γs and killer cells.

### Example B-1

### Preparation of Hybridoma H-1

### Example B-1-1

### Preparation of transformant KGFHH2

To a 0.5-ml reaction tube were added 8 µl of 25 mM magnesium chloride, 10 µl of 10xPCR buffer, one µl of 25 mM dNTP mix, one µl of 2.5 units/µl of AmpliTaq DNA polymerase, one ng of a recombinant DNA containing the base sequence in SEQ ID NO:8 prepared from a phage DNA clone and containing a DNA encoding the polypeptide in SEQ ID NO:1, and an adequate amount of a sense primer and an anti-sense primer represented by 5'-ATAGAATTCAAATGTACTTTGGCAAGCTTGAATC-3', chemically synthesized based on an amino acid sequence near the N- and C-termini of SEQ ID NO:1, and 5'-ATAAAGCTTCTAGTCTTCGTTTTGAAC-3', and the mixture solution was volumed up with sterilized distilled water to give a total volume of 100 µl. The mixture solution was in usual manner successively incubated at 94°C for one min, at 43°C for one min, and at 72°C for one min, and this sequential incubation was repeated 3 times. The resultant mixture was further successively incubated at 94°C for one min, at 60°C for one min, and at 72°C for one min, and this sequential incubation was repeated 40 times to effect PCR reaction.

The resultant PCR reaction mixture and "pCR-Script SK (+)", a plasmid vector commercialized by Stratagene Cloning Systems, California, USA, were ligated with DNA ligase to obtain a recombinant DNA which was then introduced with competent cell into "*Escherichia coli* XL-1 Blue MRF'Kan", a microorganism commercialized by Stratagene Cloning Systems, California, USA, to transform the microorganism. The transformant thus obtained was inoculated into L-broth (pH 7.2) containing 50 µg/ml ampicillin, and cultured at 37°C for 18 hours under shaking conditions, followed by centrifuging the resultant culture to collect the proliferated transformants, and isolating recombinant DNAs with conventional alkaline-SDS method. A part of the recombinant DNAs was provided, and analyzed on dideoxy method and revealing that it contained a DNA which has cleavage sites of *Eco* RI and *Hind* III at the 5'- and 3'-termini of SEQ ID NO:8, a methionine codon which initiates the polypeptide synthesis and positions in the sites corresponding to the those before and after the N- and C-termini of SEQ ID NO:8, and a TAG codon which terminates the polypeptide synthesis.

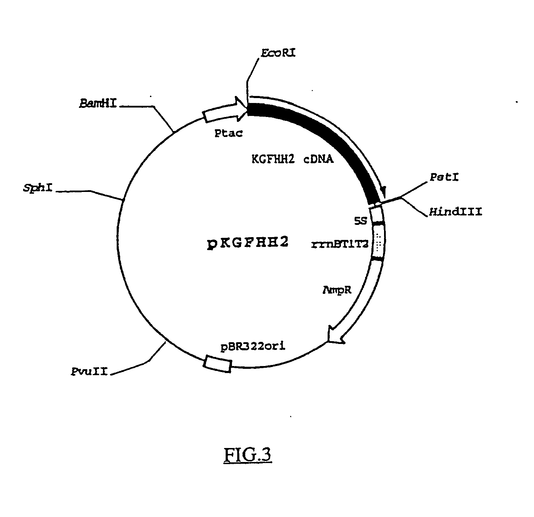

The remaining recombinant DNAs were cleaved with restriction enzymes *Eco* RI and *Hind* III, and 0.1 µg of the resultant *Eco* RI-*Hind* III DNA fragment obtained with "DNA LIGATION KIT Version 2", a DNA ligation kit commercialized by Takara Shuzo Co., Ltd., Tokyo, Japan, and 10 ng of "pKK223-3", a plasmid vector commercialized by Pharmacia LKB Biotechnology AB, Uppsala, Sweden, which had been previously cleaved with the above restriction enzymes, were ligated by incubating them at 16°C for 30 min to obtain a replicable recombinant DNA "pKGFHH2", By using competent cell method, *Escherichia coli* Y1090 strain (ATCC 37197) was transformed with the replicable recombinant DNA pKGFHH2, and the formed transformant "KGFHH2" was inoculated into L-broth (pH 7.2) containing 50 µg/ml ampicillin, and incubated at 37°C for 18 hours under shaking conditions. The resultant culture was centrifuged to collect the proliferated transformants, and a portion of which was treated with conventional SDS-alkaline method to extract the recombinant DNA pKGFHH2. As is shown in FIG.3, the analysis of dideoxy method revealed that, in the recombinant DNA pKGFHH2, the KGFHH2 cDNA which contained the base sequence in SEQ ID NO:8 was ligated to the downstream of a Tac promoter.

### Example B-1-2

### Production of polypeptide from transformant KGFHH2

An L-broth (pH 7.2) containing 50 µg/ml of ampicillin was sterilized by autoclaving, cooled to 37°C, inoculated with the transformant KGFHH2 in Example B-1-1, and incubated at the same temperature for 18 hours under shaking conditions to obtain a seed culture. An eighteen L of a fresh preparation of the same medium was placed in a 20-L jar fermenter, sterilized similarly as above, cooled to 37°C, inoculated with one v/v % of the seed culture, and cultured at the same temperature for 8 hours under aeration and agitation conditions. The resultant culture was centrifuged to collect cells which were then suspended in a mixture solution (pH 7.3) consisting of 150 mM sodium chloride, 16 mM disodium hydrogen phosphate, and 4 mM sodium dihydrogen phosphate, disrupted with ultrasonic, and centrifuged to remove cell debris to obtain a supernatant.

Ammonium sulfate was added to the supernatant up to give a concentration of 40 w/v % and dissolved to homogeneity, and the solution was centrifuged to obtain a supernatant. The supernatant was first mixed with 150 mM phosphate buffer (pH 6.6) containing 1.5 M ammonium sulfate, then fed to a column packed with "PHENYL SEPHAROSE", a product of Pharmacia LKB Biotechnology AB, Uppsala, Sweden, which had been previously equilibrated with 10 mM phosphate buffer (pH 6.6) containing 1.5 M ammonium sulfate, followed by washing the column with a fresh preparation of the same buffer, and feeding to the column a gradient buffer of ammonium sulfate ranging from 1.5 M to 0 M in 10 mM phosphate buffer (pH 6.6).

Fractions eluted at around 1.0 M ammonium sulfate were pooled, membrane filtered, dialyzed against 10 mM phosphate buffer (pH 6.5) at 4°C for 18 hours, and fed to a column packed with "DEAE 5PW", a product commercialized by Tosoh Corporation, Tokyo, Japan, which had been previously equilibrated with 10 mM phosphate buffer (pH 6.5), followed by washing the column with a fresh preparation of the same buffer, and feeding to the column a linear gradient buffer of sodium chloride ranging from 0 M to 0.2 M in 10 mM phosphate buffer (pH 6.5) while collecting fractions eluting at 0.05 M sodium chloride.

Thereafter, the fractions were concentrated with a membrane and fed to a column packed with "SUPER DEX 75", a product of Pharmacia LKB Biotechnology AB, Uppsala, Sweden, which had been equilibrated with phosphate buffered saline (hereinafter abbreviated as "PBS"), followed by feeding to the column a fresh preparation of PBS to collect fractions corresponding to about 18,500 daltons. Thus, an aqueous solution containing about 5.2 mg of a purified protein was obtained. The total yield throughout the purification was about 10%.

The purified protein was analyzed and found that it had the following physicochemical properties: When electrophoresed in SDS-polyacrylamide gel under reducing conditions, the purified protein appeared as a main protein band having an IFN-γ inducibility at a position corresponding to 18,500±3,000 daltons, while giving a pI of 4.9±1.0 on chromatofocusing. The amino acid sequence containing N-terminus of the purified protein had the amino acid sequence in SEQ ID NO:9 equal to that in SEQ ID NO:1 where methionine was coupled to its N-terminus.

### Example B-1-3

### Preparation of hybridoma H-1

BALB/c mice, 10-week-old, were intraperitoneally injected with 20 µg/mouse of a purified polypeptide, obtained by the method in Example B-1-2, together with a complete Freund's adjuvant. The mice were further injected twice with the same dose at an interval of 2 weeks and intravenously injected with the same dose one week after the final injection, and their spleens were extracted and suspended to obtain a cell suspension.

The spleen cells and SP2/O-Ag14 cells from mouse myeloma (ATCC CRL 1581) were suspended in RPMI 1640 medium (pH 7.2) preheated to 37°C at cell densities of 3x10⁴ cells/ml and 1x10⁴ cells/ml, respectively, and centrifuged to collect sediment. One ml of a serum-free RPMI 1640 medium (pH 7.2), containing 50 w/v % polyethylene glycol with an average molecular weight of 1,500 daltons, was added drop-wise to the sediment over a min, and the mixture was incubated at 37°C for a min, followed by adding drop-wise to the mixture a serum-free RPMI 1640 medium (pH 7.2) up to give a total volume of 50 ml, centrifuging the mixture, and collecting the formed sediment. The sediment thus obtained was suspended in HAT medium, distributed to 96-well microplates in an amount of 200 µl/well, and incubated at 37°C for one week, followed by selecting hybridomas.

The amount of antibodies secreted in the supernatant in each well was assayed on enzyme immunoassay based on the immunoreaction of the antibodies and a purified polypeptide, obtained by the method in Example B-1-2, and hybridomas capable of producing antibodies, which strongly react with the purified polypeptide, were selected. A cloned hybridoma H-1 cell capable of producing the present monoclonal antibody was in usual manner obtained by repeatedly treating these hybridomas with limiting dilution.

### Example B-2

### Preparation of monoclonal antibody H-1mAb and

### analysis on the Western blot technique

### Example B-2-1

### Preparation of monoclonal antibody H-1mAb

Hybridoma H-1 cells obtained by the method in Example B-1-3 were suspended in RPMI 1640 medium (pH 7.2) supplemented with 5 v/v % calf serum to give a cell density of about 1x10⁶ cells/ml, and incubated in an incubator at 37°C under 5 v/v % CO₂ conditions while scaling up the culture. When the cell density of the culture reached to a prescribed level, 1x10⁷ cells/mouse of the proliferated hybridoma H-1 cells were intraperitoneally injected to BALB/c mice, 8-week-old, which had been previously intraperitoneally injected with 0.5 ml/mouse of pristane, followed by feeding the mice in usual manner for one week.

From the mice ascites were collected, diluted with PBS by 3 times, mixed with ammonium sulfate to give a saturation degree of 50 w/v %, allowed to stand at 4°C for 24 hours, and centrifuged to collect sediment. The sediment was dialyzed against an aqueous solution of 20 mM potassium dihydrogen phosphate (pH 6.7) at 4°C overnight, and fed to a column of hydroxyapatite which had been previously equilibrated with a fresh preparation of the same aqueous solution, followed by feeding to the column a linear gradient potassium dihydrogen phosphate buffer (pH 6.7) ranging from 20 mM to 300 mM to obtain an aqueous solution containing the present monoclonal antibody H-1mAb. The yield was about 5 mg per mouse. Conventional analysis revealed that the antibody belongs to the class of IgG₁.

### Example B-2-2

### Analysis on the Western blot technique

One µg of a purified polypeptide, obtained by the method in Example B-1-2, was added to a mixture solution consisting of 100 mg dithiothreitol, 0.5 ml of a 10 w/v % aqueous SDS solution, and one ml of glycerol, and the mixture was incubated at 37°C for one hour and electrophoresed in SDS-polyacrylamide gel. The resultant gel was in usual manner transferred to a nitrocellulose membrane which was then soaked in a culture supernatant of hybridoma H-1 cells for one hour, and washed with 50 mM Tris-HCl buffer (pH 7.5) containing 0.05 v/v % tween 20 to remove excessive amounts of antibodies. The membrane was further soaked for one hour in PBS containing an anti-mouse Ig antibody prepared from rabbits to effect immunoreaction, washed with 50 mM Tris-HCl buffer (pH 7.5) containing 0.05 v/v % tween 20, and soaked in 50 mM Tris-HCl buffer (pH 7.5) containing 0.005 v/v % hydrogen peroxide and 0.3 mg/ml 3,3'-diaminobenzidine to effect coloration.

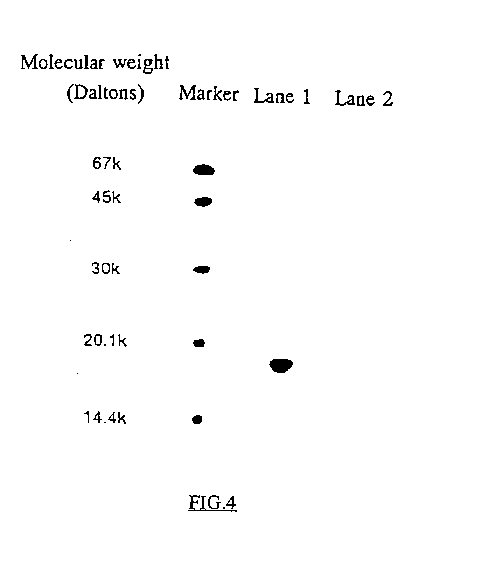

As a control, a system using a recombinant human interleukin 12 in place of the purified polypeptide was provided, and similarly treated as above. Calf serum albumin (MW=67,000 daltons), ovalbumin (MW=45,000 daltons), carbonic anhydrase (MW=30,000 daltons), trypsin inhibitor (MW=20,100 daltons), and α-lactalbumin (MW=14,400 daltons) were used as a marker protein. These results were in FIG.4.

As is evident from FIG.4, the monoclonal antibody H-1mAb specifically reacted with the purified polypeptide (Lane 1) obtained by the method in Example B-1-2, but did not with the human interleukin 12 (Lane 2). This evidences that the present monoclonal antibody specifically reacts with the polypeptide with a specific amino acid sequence.

### Example B-3

### Preparation of hybridoma H-2 and monoclonal antibody H-2mAb

Hybridoma H-2, a monoclonal antibody, was similarly prepared by the method in Example B-2-1 except that P3-X63-Ag8 cells (ATCC TIB9) were used in place of the SP/O-14Ag cells.

### Example B-3-2

### Preparation of monoclonal antibody H-2mAb

The hybridoma H-2 in Example B-3-1 was cultured similarly as in Example B-2-1, and the culture was purified to obtain an about 5.6 mg of monoclonal antibody H-2mAb per BALB/c mouse. Conventional analysis revealed that the monoclonal antibody belongs to the class of IgM, and it specifically reacted with a purified polypeptide obtained by the method in Example B-1-2 when analyzed on Western blotting technique similarly as in Example B-2-2.

### Example B-4

### Purification of polypeptide on immunoaffinity chromatography

### Example B-4-1

### Preparation of gel for immunoaffinity chromatograph

Eighty mg of monoclonal antibody H-1mAb, obtained by the method in Example B-2-1, was weighed and dialyzed against 0.1 M borate buffer (pH 8.5) containing 0.5 M sodium chloride at 4°C overnight. Four g of "CNBr-activated Sepharose 4B", a water-insoluble carrier commercialized by Pharmacia LKB Biotechnology AB, Uppsala, Sweden, was swelled with one mM of aqueous chloric acid solution, successively washed with a fresh preparation of the same buffer and 0.1 M borate buffer (pH 8.5) containing 0.5 M sodium chloride, admixed with an about 10 ml of the aqueous monoclonal antibody solution obtained in the above, and successively incubated at ambient temperature and at 4°C overnight under gentle stirring conditions. Thereafter, the resultant gel was successively washed with one M aqueous ethanol amine solution (pH 8.0), 0.1 M borate buffer (pH 8.5) containing 0.5 M sodium chloride, and 0.1 M acetate buffer (pH 4.0), and these washing steps were repeated 5 times. Finally, the gel was washed with PBS to obtain a gel for immunoaffinity chromatography. Conventional analysis revealed that about 6 mg monoclonal antibody H-1mAb linked to one ml of the gel.

### Example B-4-2

### Purification of polypeptide on immunoaffinity chromatography

Ten ml of the gel for immunoaffinity chromatography in Example B-4-1 was packed in a plastic cylindrical column, washed with PBS, and fed with 10 ml of a Phenyl Sepharose eluted fraction containing about 0.1 mg/ml of the polypeptide obtained by the method in Example B-1-2. The column was washed with a fresh preparation of PBS, and fed with 0.1 M glycine-HCl buffer (pH 2.5) containing one M sodium chloride to collect fractions with an IFN-γ inducing activity. The fractions were pooled, dialyzed against PBS at 4°C overnight, concentrated, and assayed for the IFN-γ inducing activity and the protein content and revealing that this purification procedure yielded a purified polypeptide with a purity of 95 w/w % or higher in a yield of about 100%.

### Example B-5

### Detection of polypeptide on enzyme immunoassay

Rabbits were in usual manner immunized with a purified polypeptide obtained by the method in Example B-1-2, and collected their blood. Immunoglobulin G antibody was isolated from the blood, and dissolved in PBS to give a concentration of 20 µg/ml, and the solution was distributed into 96-well microplates in an amount of 100 µl/well. The microplates were incubated at ambient temperature for 3 hours, followed by removing solutions containing IgG from the microplates, adding PBS containing one w/v % calf serum albumin to the microplates in an amount of 200 µl/well, and allowing them to stand at 4°C overnight.

Phosphate buffered saline was removed from the microplates which were then washed with PBS containing 0.05 v/v % tween 20, and injected with 100 µl/well of a solution prepared by appropriately diluting a purified polypeptide, obtained by the method in Example B-1-2, with PBS containing 0.5 w/v % calf serum albumin, followed by reacting the mixture solution at ambient temperature for 2 hours under shaking conditions. The microplates were washed with PBS containing 0.05 v/v % tween 20, and injected with 100 µl/well of a solution containing a monoclonal antibody H-1mAb labelled with biotin, followed by reacting the mixture solution at ambient temperature for 2 hours under shaking conditions, washing the microplates with PBS containing 0.05 v/v % tween 20, injecting with 100 µl/well of a solution containing a complex of horseradish peroxidase and streptoavidin, and further reacting the resultant mixture at ambient temperature for 2 hours under shaking conditions. Then, the microplates were washed with PBS containing 0.05 v/v % tween 20, and the activity of the horseradish peroxidase linked to the purified polypeptide was measured for absorbance at a wavelength of 492 nm using o-phenylenediamine as a substrate. The results were in Table 6.

**Table 6**

| Concentration of polypeptide (pg/ml) | Absorbance at 492 nm* | Relative error (%) |
|---|---|---|
| 1,000 | 1.51±0.05 | 3.3 |
| 500 | 0.93±0.05 | 5.4 |
| 250 | 0.55±0.03 | 5.5 |
| 100 | 0.25±0.02 | 8.0 |
| 50 | 0.137±0.007 | 5.1 |
| 25 | 0.080±0.007 | 8.8 |
| 0 | 0.024±0.007 | - |

| | | |
|---|---|---|
| Note : The symbol "*" means a statistical value of triplet. | | |

As is evident from the results in Table 6, the detection method according to the present invention accurately assays the polypeptide in the range of about 50-1,000 pg/ml.

### Example B-6

### Detection of polypeptide on radioimmunoassay

Rabbits were in usual manner immunized with a purified polypeptide obtained by the method in Example B-1-2, and collected their blood, followed by isolating IgG antibody. The antibody was in usual manner adsorbed on polystyrene beads for radioimmunoassay, and allowed to stand in PBS containing 2 w/v % calf serum albumin at 4°C overnight to obtain an immobilized antibody.

One bead was placed in a test tube, soaked in 0.2 ml of a solution prepared by diluting a purified polypeptide, obtained by the method in Example B-1-2, with PBS containing 0.5 w/v % calf serum albumin, and allowed to stand at 4°C for 4 hours. Then, the bead was washed with PBS containing 0.05 v/v % tween 20 and 0.5 w/v % calf serum albumin, soaked in 0.2 ml (1x10⁵ cpm) of a solution containing a monoclonal antibody H-2mAb, obtained by the method in Example B-3-2 and labelled with ¹²⁵I, and allowed to stand at 4°C overnight. After removing an excessive amount of ¹²⁵I-labelled antibody, the bead was washed with PBS containing 0.05 v/v % tween 20 and 0.5 w/v % calf serum albumin, followed by counting the radioactivity of the bead on a gamma-counter. The results were in Table 7.

**Table 7**

| Concentration of polypeptide (pg/ml) | Count* (cpm) | Relative error (%) |
|---|---|---|
| 1,000.0 | 6,900±200 | 2.9 |
| 500.0 | 4,100±20 | 0.5 |
| 250.0 | 2,390±50 | 2.1 |
| 125.0 | 1,590±70 | 4.4 |
| 62.5 | 880±10 | 1.1 |
| 0 | 700±20 | - |

| | | |
|---|---|---|
| Note : The symbol "*" means a statistical value of triplet. | | |

As is evident from the results in Table 7, the present detection method accurately assays the polypeptide in the range of about 100-1,000 pg/ml.

### Example C-1-Reference Example

### Solution

A polypeptide, obtained by the method in Example B-1-2, was dissolved in physiological saline containing one w/v % human serum albumin as a stabilizer to obtain a one mg/ml polypeptide solution which was then sterilized by membrane filter to obtain a solution.

The product with a satisfactory stability can be used as an injection, ophthalmic solution, and collunarium in the treatment and/or the prevention of susceptive diseases such as malignant tumors, viral diseases, bacterial infectious diseases, and immune diseases.

### Example C-2 - Reference Example

### Dry injection

A polypeptide, obtained by the method in Example B-1-2, was dissolved in 100 ml physiological saline containing one w/v % purified gelatin as a stabilizer, and the solution was in usual manner sterilized with a membrane filter. One ml aliquots of the sterilized solution were distributed to vials, lyophilized, and cap sealed.

The product with a satisfactory stability can be used as a dry injection for treating and/or preventing susceptive diseases such as malignant tumors, viral diseases, bacterial diseases, and immune diseases.

### Example C-3 Reference Example

### Ointment

"HI-BIS-WAKO 104", a carboxyvinyl polymer commercialized by Wako Pure Chemicals, Tokyo, Japan, and a purified trehalose were dissolved in distilled water to give concentrations of 1.4 w/w % and 2.0 w/w %, respectively, and a polypeptide obtained by the method in Example B-1-2 was dissolved to homogeneity in the solution, followed by adjusting the pH of the resultant solution to pH 7.2 to obtain a paste containing about one mg/g of the polypeptide.

The product with a satisfactory spreadability and stability can be used as an ointment for treating and/or preventing susceptive diseases such as malignant tumors, viral diseases, bacterial infectious diseases, and immune diseases. Example C-4 Reference Example

### Tablet

A polypeptide, obtained by the method in Example B-1-2, and LUMIN, i.e. [bis-4-(1-ethylquinoline)][γ-4'-(1-ethylquinoline] pentamethionine cyanine, as a cell activator were mixed to homogeneity with "FINETOSE^{®}", an anhydrous crystalline α-maltose commercialized by Hayashibara Co., Ltd., Okayama, Japan, and the mixture was in usual manner tabletted by a tabletting machine to obtain tablets, about 200 mg weight each, containing the polypeptide and the LUMIN, about one mg each.

The product, having a satisfactory swallowing ability, stability, and cell activating activity, can be used as a tablet for treating and/or preventing susceptive diseases such as malignant tumors, viral diseases, bacterial infectious diseases, and immune diseases.

### Example C-5 - Reference Example

### Adoptive immunotherapeutic agent

Mononuclear cells were isolated from peripheral blood of a patient with malignant lymphoma, suspended in RPMI 1640 medium (pH 7.2) which was supplemented with 10 v/v % human AB serum and preheated to 37°C to give a cell density of about 1x10⁶ cells/ml, and mixed with about 1.0 µg/ml of a polypeptide, obtained by the method in Example B-1-2, and about 100 units/ml of a recombinant human interleukin 2, followed by incubating the resultant in a 5 v/v % CO₂ incubator at 37°C for one week, and centrifuging the resultant culture to collect LAK cells.

The LAK cells thus obtained exhibit a strong cytotoxicity on lymphoma cells when introduced into the body of the donor patient, and exert a higher cytotoxicity than that attained by the adoptive immunotherapy using interleukin 2 alone. Cytotoxic T-cells, obtained by similarly treating lymphocytes invaded into tumor tissues from the patient, in place of the above lymphocytes, was injected into the donor patient and resulting in an exertion of the similar effect attained by the LAK cells. The adoptive immunotherapeutic agent can be arbitrarily used to treat solid malignant tumors such as renal cancer, malignant melanoma, colonic cancer, rectal cancer, and lung caner.

The present description is based on the finding of a novel polypeptide which induces the IFN-γ production by immunocompetent cells. The polypeptide is a substance which has a partially or totally revealed amino acid sequence, and a stable activity of inducing IFN-γ production by immunocompetent cells.

The polypeptide has a strong IFN-γ inducibility so that it can induce a desired amount of IFN-γ production with only a small amount. The polypeptide dose not cause serious side effects even when administered to in a relatively-high dose because it only has an extremely-low toxicity. Therefore, the present polypeptide has an advantage that it promptly induces a desired amount of IFN-γ production without strictly controlling the dose.

The present monoclonal antibody specifically reacts with the polypeptide, and is widely used in the purification and the detection of the polypeptide. The antibody is prepared in a desired amount by using hybridomas.

The present agent for susceptive diseases exerts a satisfactory effect in the treatment and/or the prevention of susceptive diseases such as malignant tumors, viral diseases, bacterial infectious diseases, and immune diseases. Furthermore, the agent has an activity of enhancing the cytotoxicity by killer cells or of inducing the formation of killer cells, and exerts a significant effect in the treatment of serious diseases such as malignant tumors.

Thus, the present invention is a significant invention which has a remarkable effect and gives a great contribution to this field.

## Claims

1. A composition for specifically reacting with a polypeptide of human origin, which comprises as an effective ingredient a monoclonal antibody recognizing the polypeptide along with a carrier of the monoclonal antibody; said polypeptide inducing the IFN-gamma production by immunocompetent cells and comprising the amino acid sequence of SEQ ID No: 1 (where the symbol "Xaa" means "isoleucine" or "threonine").

2. An in vitro method for specifically reacting a polypeptide of human origin with a monoclonal antibody, which comprises a step of contacting the polypeptide with a monoclonal antibody recognizing the polypeptide; said polypeptide inducing the IFN-gamma production by immunocompetent cells and comprising the amino acid sequence of SEQ ID No: 1 (where the symbol "Xaa" means "isoleucine" or "threonine").

## Patentansprüche

1. Rezeptur, speziell zur Reaktion mit einem Polypeptid menschlichen Ursprungs, die als einen wirkenden Mischungsbestandteil einen monoklonalen Antikörper, der das Polypeptid erkennt, zusammen mit einem Träger des monoklonalen Antikörpers aufweist; wobei das Polypeptid die Interferongamma Produktion durch Immunzellen induziert und die Aminosäuresequenz SEQ ID Nr. 1 (bei der das Symbol "Xaa" "Isoleucin" oder "Threonin" bedeutet) aufweist.

2. In vitro Verfahren, speziell zur Reaktion eines Polypeptids menschlichen Ursprungs mit einem monoklonalen Antikörper, das einen Schritt des Kontaktierens des Polypeptids mit einen monoklonalen Antikörper, der das Polypeptid erkennt, aufweist; wobei das Polypeptid die Interferon-gamma Produktion durch Immunzellen induziert und die Aminosäuresequenz SEQ ID Nr. 1 (bei der das Symbol "Xaa" "Isoleucin" oder "Threonin" bedeutet) aufweist.

## Revendications

1. Composition destinée à réagir spécifiquement avec un polypeptide d'origine humaine, qui comprend comme ingrédient efficace un anticorps monoclonal reconnaissant le polypeptide avec un porteur de l'anticorps monoclonal ; ledit polypeptide induisant la production de gamma IFN par des cellules immunocompétentes et comprenant la séquence d'acides aminés de SEQ ID No: 1 (où le symbole « Xaa » signifie « isoleucine » ou « thréonine »).

2. Procédé in vitro destiné à faire réagir spécifiquement un polypeptide d'origine humaine avec un anticorps monoclonal, qui comprend une étape consistant à mettre en contact le polypeptide avec un anticorps monoclonal reconnaissant le polypeptide ; ledit polypeptide induisant la production de gamma IFN par des cellules immunocompétentes et comprenant la séquence d'acides aminés de SEQ ID No: 1 (où le symbole « Xaa » signifie « isoleucine » ou « thréonine »).
